# EUROPEAN PATENT APPLICATION

(11) **EP 4 571 759 A1**
(43) Date of publication of application: **18.06.2025**
(21) Application number: 23215773.5
(22) Date of filing: 12.12.2023
(51) Int. Cl.: G16H 20/30, G16H 20/70

(54) **METHOD AND SYSTEM FOR IMPROVING USER PERFORMANCE OF AT LEAST ONE USER AT A DEFINED POINT IN TIME IN THE FUTURE**

(71) Applicant: Koa Health Digital Solutions S.L.U., 08018 Barcelona (ES)
(72) Inventor: Harrison, Oliver, 08018 Barcelona (ES); Garcia i Tormo, Albert, 08018 Barcelona (ES); Matic, Aleksandar, 08018 Barcelona (ES); Johnston, Tania, 08018 Barcelona (ES); Hemmings, Nicola, Barcelona, 08018 (GB)
(74) Representative: Herrmann, Uwe

(57) **Abstract**

The present invention pertains to a computer-implemented method and a device for improving user performance of at least one user at at least one defined point in time in the future. According to the invention, a predicted numerical user performance score at the defined point in time (e.g a performance score of 85 %) is calculated and compared to a desired user performance (e.g. a performance of at least 90%). If the predicted user performance score falls short of the desired user score, the invention provides a customized program for the individual user comprising activities to interactively support the user in achieving their desired performance level at the defined point in time.

## Description

The present invention pertains to a method and system for improving user performance of at least one user at a defined point in time in the future.

In everybody's life, certain instances require relatively high levels of user performance, being it carefully and concentratedly executing a manufacturing step, focussing while performing a surgery, sitting examinations during one's education or participating in a competition. These instances are often scheduled based on external circumstances and not scheduled taking into account the individual characteristics of the participants, for example an individual's chronotype of individual fatigue level. This hampers performance of participants in practice and often poses an obstacle to participants achieving their possible optimal performance.

The present invention strives to overcome these issues and assist users in achieving their desired performance level at a given point in time in the future.

For example, the present invention pertains to a computer-implemented method and a device for improving user performance of at least one user at at least one defined point in time in the future. According to the invention, a predicted numerical user performance score at the defined point in time (e.g a performance score of 85 %) can be calculated and compared to a desired user performance (e.g. a performance of at least 90%). Thus, the present invention can offer the advantage of relatively accurately gauging user performance at the defined point in time in the future based on a case wherein no intervention to improve the user performance is made.

If the predicted user performance score falls short of the desired user performance score, the invention can provide a customized program for the individual user comprising recommendations such as activities to interactively support the user in achieving their desired performance level at the defined point in time.

For example, the invention can prompt the user to perform additional activities, such as resting or a relaxation exercise, or to re-schedule planned activities, for example to wake-up an hour earlier in the morning to feel more awake at the desired point in time, e.g. an important meeting.

In the following, background information on the present invention is provided.

People carry out different activities of different types throughout the day. For instance, in a day, one may run, read a book, play the piano or think about how to solve a puzzle. Despite the different nature of these activities (physical or cognitive), they all demand a certain level of effort and a certain performance from the person performing them. This effort causes the person to fatigue (physically and/or cognitively), which hinders them in performing the activity to the best of their ability. To recover from fatigue, the person needs to stop doing the activity and rest.

Not all individuals build up and recover from fatigue in the same way, just like not all activities are equally intense or demanding. However, since fatigue accumulates for as long as the person carries on with an activity, it is necessary to take a break from any activity, once a certain level of fatigue has built up. There is thus a common recurrent pattern that applies to all activities and all individuals: *activity* → *fatigue* → *rest* → *activity→ [...]*.

High levels of fatigue hinder individuals in performing tasks: the higher the fatigue, the lower the individual can perform. Physical activities illustrate fatigue well: e.g. a person could start jogging at 15 km/h and, after 15 min of jogging, the person needs to lower the speed to 12 km/h because they are *getting tired*; in other words, the accumulated fatigue prevents this person to continue performing the activity at the desired level of performance. Note that fatigue does not necessarily prevent the person from performing the activity unless the fatigue is very high, it only constrains the optimal performance that this person can achieve (in this example, after jogging for 15 min at 15km/h, the person can continue jogging but at lower speed); also note that for certain activities or certain individuals, the person may not realise the accumulated fatigue and their current under-performance. Physical examples are easy to understand and relate to, but it is analogous for cognitive activities such as an exam or a meeting.

Continuing with the jogging example, if the person wants to keep jogging for ten more minutes at 15 km/h, they need to perform a resting activity to decrease the fatigue level (recovery) and be able to perform again at the desired level. In this case, a suitable resting activity may be walking and stretching for 5 min.

The most fundamental recovery activity is sleep. Humans can recover both physically and mentally by sleeping. The amount of recovery during sleep is defined not only by the total number of hours of sleep (sleep quantity), but also by how well the person sleeps (sleep quality). Sleep quality is usually evaluated by analysing the amount of time slept at each of the sleep stages; typically three different sleep stages are considered for evaluating sleep quality (Rapid Eye Movement - REM, deep sleep and light sleep, and awake but this is not a sleep stage by itself). During sleep, the person cycles through the stages; depending on the amount of time spent at each stage (i.e. the sleep quality), a different degree of recovery will be achieved. REM is generally associated with cognitive recovery and deep sleep is associated with physical recovery.

Sleep and sleep quality in particular, have been widely studied in the literature. Studies indicate that sleeping in a fresh, dark and quiet environment is usually associated with a higher sleep quality; likewise, alcohol or heavy meals right before sleeping are associated with lower sleep quality. Sleeping seven hours per day is the general recommendation for adults. However, it is worth emphasising that all these are only general guidelines-there exist differences between individuals and, for instance, an individual may need to sleep nine hours every day and another individual may only need to sleep five.

Even though sleep is very effective at recovering from both physical and cognitive fatigue, if the accumulated fatigue is high enough (or if the quality and/or quantity of sleep is not high enough), a single period of sleep may not be enough to fully recover. The accumulated fatigue is hence passed to the next day (or days) and it may constrain the performance just as if the fatigue had been accumulated in the same day. Multiple days of recovery, as well as multiple recovery activities, may be necessary to fully recover in some cases.

It is not always advisable or feasible to rely on sleep for recovery. Sometimes it is advantageous to rely on a different recovery activity, of the same nature or of different nature than the demanding activity. For instance, after a long exam (high-demanding cognitive activity), a mild swim (low-intensity physical activity) can accelerate the cognitive recovery process. Similarly, performing specific activities right before (or right after) another activity, can help lower the speed at which fatigue is built upon or increase the recovery speed; for instance, a warming-up activity prior to an intense run can help the individual reduce the fatigue built up during the intense run, and a stretching activity can help speed up muscles recovery after a workout session. Specific warming-up and cooling-down activities are not only advisable for physical activities, but for cognitive activities as well. For instance, a chess player, before starting a competition match, may perform a warming-up routine that consists in rapidly finding the best move in a set of given chess games.

All activities, including resting ones, involve an energy expenditure. Resting activities such as sitting on a chair involve energy expenditure because keeping the body warm requires a significant amount of energy. The energy is obtained from food. Diets also play a role in building up and recovering from fatigue. Without the proper nutrients, the body and the mind can neither properly function nor properly recover. Professional athletes follow special diets to optimise their recovery after training and events, so that they recover faster and are ready to train again and perform better every time. Business people such as CEOs may also follow nutrition plans, not only to keep a balanced weight and stay healthy, but also to perform better in meetings and be able to handle high peaks of work.

Nutrition plans may include advice for both food and beverages, not only relating to what to eat and drink in general, but also specifying quantities to ingest (how much to eat, including specific advice for key substances such as caffeine or fibre), the time in the day and how many times per day to ingest (when), or about how to ingest (e.g. sitting on a chair in a living room, while walking, on the desk in front of the computer). Often nutrition plans are combined with other activities such as physical exercise. For instance, a weight-loss diet is often recommended in combination with walks or mild runs, to be more effective. All processes in the body are ultimately interrelated and therefore, physical performance, cognitive performance, recovery and diet are not independent from each other. Because of this, when attempting to optimise the performance for a certain activity or set of activities, a multidisciplinary advice including physical activities, cognitive activities, diet and rest is often given.

The human body has an internal biological clock (circadian clock) that regulates internal biological processes such as sleep. The period of this clock is about 24h, which approximately coincides with one day (one rotation of the Earth over its axis, thereby generating a morning, a noon, an afternoon, an evening and a night). Consequently all functions regulated by the circadian clock follow a recurrent 24-hour behaviour including two distinct parts, a high-activation part and a low-activation part. Using the example of sleep, as this is a circadian-regulated process, there are moments of the day wherein the person feels very sleepy and it is very likely that they can sleep (usually evening and night, when sleepiness is high) and also there are moments in the day wherein the person feels very awake and it is unlikely that they can sleep (usually morning, noon and late afternoon, when sleepiness is low). The transition between the high and the low parts is smooth, although, within each part, there may be local peaks or valleys (e.g. after lunch, the sleepiness has a local peak).

Although generally all the functions regulated by the circadian clock follow a recurrent 24-hour period, the body regulates them with a different phase. The phases are set to maximise performance and recovery throughout the day. For instance, sleep has its peak close to midnight, to devote the hours with no sunlight to recovery (i.e. the hours when it is most difficult to move around); alertness, however, has its peak close to noon, when there is maximum sunlight and one needs to perform activities such as walking on the mountains without tripping to collect food. This natural oscillation of the body-internal processes is known as the circadian rhythm. More examples of processes regulated by the circadian rhythm are coordination, body temperature and reaction time.

Whilst there are differences in the circadian clock between individuals (e.g. the circadian clock's period of one person may be 24,1h and the circadian clock of another person may be 25,3h), within the same individual, all circadian-regulated processes are driven by very same clock and therefore the order in which the peak of each function is achieved every day is always the same.

The period of the circadian clock approximately coincides with an Earth's day, but there is some mismatch between the period of a person's circadian clock and the Earth's day. The mismatch can be small (in the range of seconds) but it can also be large (like an hour). Despite the frequency mismatch between the two clocks (circadian clock and Earth's day), they do not lose synchrony unless there is a major disruption; in other words, if a person follows a daylight-based schedule (i.e. sleeping during the night and being awake during the day), then the circadian clock remains synchronised with the Earth's day. The mechanism that keeps the synchrony is sleep.

Sleep is a mechanism that triggers recovery in all internal processes; when a person falls asleep, recovery begins. Despite sleep being circadian-controlled, one can try to sleep at a different time and advance or delay the start of the recovery accordingly. In general, if the delay/advancement is about two or three hours or less (namely, X hours), then the person is able to properly sleep and recover (this is, to get enough sleep time and with enough quality); then, the next day, they are fully recovered X hours later/earlier than the day before. That is also why, despite the mismatch between the circadian clock's period and length of an Earth's day, the circadian clock tracks the Earth's day without losing synchrony. However, if the delay/advancement is too large (typically three hours or more), then the processes do not fully reset during sleep and, when the person wakes up after sleeping, their schedule (i.e. the activities that the person does during the day) is no longer in phase with the circadian-controlled processes.

The circadian clock generally behaves similarly to a Phase Locked Loop (PLL). A PLL is a system with a local oscillator that adjusts its phase and its frequency so that the local oscillator (output signal) tracks an input signal. When no input signal is provided, the local oscillator oscillates at its free-running frequency; when the input is supplied with a periodic (or almost periodic) signal whose frequency is close to the free-running frequency (specifically, the frequency of the input signal is within the pull-in range), then the PLL adjusts its frequency and phase so that the output signal tracks the frequency and the phase of the input signal (the PLL is locked). When the PLL is locked and the input slightly changes (a change in the frequency or in the phase), the PLL is able to track such changes and the output keeps tracking the input (hold-in range and bandwidth); however, if the changes are too large (i.e. beyond the PLL's tracking capabilities), then the PLL unlocks and stops tracking the input signal.

The circadian-rhythm behaves in a similar way; the local oscillator is the circadian clock with its own free-running frequency (e.g. 24,9h) and the input signal is the day-night cycle (an Earth's day, namely 24,0h), which is supplied to the human body mainly through the eyes (bright light during the day and darkness, or absence of light, during the night).

When a person follows a regular day-based routine (e.g. sleeping during the night, awake during the day), the circadian clock is locked to the day-night cycle and thus it oscillates at 24,0h and with the same phase (e.g. sleepiness is high during the evening and night, low during the morning and afternoon). Small differences like going to sleep 1h earlier/later than the day before are within the hold-in range and hence the circadian clock remains locked to the day-night cycle. However, a large difference like a sudden phase shift of 6h in the day-night cycle (caused by e.g. long-distance travelling by plane) is beyond tracking capabilities of the circadian system (the PLL) and the phase of the circadian clock (oscillator) cannot be adjusted accordingly; as a result, the circadian clock (output signal) no longer tracks the day-night cycle (input signal); in particular its phase, since the long-distance trip results in the circadian clock having a phase offset with respect to the new (destination's) day-night cycle. An example of a consequence of this mismatch is a high alertness and a low sleepiness during the night, which results in the person not being able to sleep during the night and feeling very sleepy during the day at the destination; such mismatch between the local daylight time circadian clock and the daylight is known as *jet lag.*

As mentioned above, adjustments up to around 2h can be followed in a single day without requiring special actions (in other words, such changes are within the tracking capabilities of the circadian system). This means that the circadian clock can track them. Small adjustments are very frequent in everyday life, to e.g. watch a movie in the evening on TV or join a mountain hike early in the morning. As long as the small adjustments keep on cancelling on each other (in other words, that the combination of a number of consecutive small adjustments does not result in a large adjustment), usually no special recommendations are needed to deal with them.

When an individual needs to perform activities outside the regular schedule, a large adjustment may be necessary, especially if the out-of-schedule activities extend a few days or more. This is typically the case in shift workers and after long-distance flights; in both cases, the sleep scheduling (and consequently all the circadian-controlled processes) needs to shift by several hours at once, and the new schedule remains in place for at least several days. Such large adjustments cannot be handled at once; instead, they need to be split into a sequence of small adjustments, which extend over a number of days. For long-distance trips, adaptation periods of up to a week are frequent.

The main mechanism for adjusting the circadian rhythm is light (and absence of light, i.e. darkness) and, in particular, blue light. Blue light corresponds to a specific wavelength range, typically between 450 and 495 nanometres, belonging to the visible range; blue light is emitted by the Sun (natural light) and also by LEDs, LASERs and other artificial light sources (artificial light). Whilst light is not the only mechanism to modify the circadian clock, it is the one that has the highest influence. Popular recommendations to help dealing and even preventing jet-lag include a light-related aspect (avoid sunlight, get sunlight, use dark sunglasses if you need to go out, etc.). Besides the light-related recommendations, another way to adjust the circadian rhythm is to ingest stimulants like caffeine or theanine. These can temporarily increase the alertness and reduce the sleepiness, thereby allowing the person to fall asleep later, triggering the recovery process later than in the previous day. Less common but still frequent is to ingest compounds like melatonin, to reduce alertness and increase sleepiness. There are also medical-prescribed drugs which achieve a stronger effect (to either stay awake or fall asleep), but these are not for everyday use.

Note that large adjustments are both for advancing and delaying the circadian clock. In the case of a long-distance flight, it may take several days to adapt to the new time zone when arriving at the destination; but it might also be the case when flying back to one's local time zone.

Inferring the current state of circadian-controlled processes (an example of an actual / individual circadian phase state of a user): All circadian-controlled processes are controlled by the same clock (circadian clock) and, for each individual, the various phase differences between them are constant. Consequently, if inferring the current state of a circadian-based process, the other phases can be derived from the first one. It is necessary, however, to know the time difference between the different phases (tailored to the individual or generalised). A possible way for inferring the current state of the circadian clock is to monitor the sleep patterns over a certain period of time (typically at least a week).

Furthermore, user chronotype can be relevant. Chronotype refers to the individual differences in activity and alertness at different moments of the day. Morning-type people naturally wake up and fall asleep earlier than evening-type people, which results in different activity schedules throughout the day. Whilst chronotypes are partly based on genetics, they are also influenced by factors like age, gender and conscious actions such as the work schedule and light exposure. Knowing one's chronotype is relevant when planning activities where performance is key and also when trying to mitigate or prevent jet-lag.

Chronotype has been traditionally assessed mainly through self-report questionnaires, such as the Morningness-Eveningness Questionnaire. In the present invention, chronotype can also be inferred based on activity data.

When measuring / predicting user performance, two elements can be relevant, base condition and readiness. In other words, the performance that one achieves at a specific event / activity is generally determined by two main aspects: the base condition and the readiness.

For the sake of simplicity, this is illustrated by an example based on a physical activity: running a 100-metre dash in a competition. The base condition relates to the physical condition of the athlete; this defines the theoretical maximum, i.e. how fast the athlete could run based on the strength of their muscles (other relevant factors such as the running technique have been omitted in this example). The base condition can be trained and improved: the more the athlete trains prior to the competition, the more strength they will have in their legs and thus the faster they can run at the competition. However, an athlete with a certain base condition, may or may not perform at their best at the competition. In other words, at the competition, the athlete may perform at any level between 0% and 100% of their base condition.

For instance, if the athlete keeps training very hard until the day before the event, at the moment of starting the competition the fatigue accumulated during the recent training sessions will prevent the athlete from performing at 100% and may only perform at 60% (underperformance due to overtraining). However, if during the last seven days prior to the competition, the athlete engages only in tune-up exercises (thereby keeping the base fitness whilst minimising fatigue), then they may perform at 100% (optimum performance given their base condition). The athlete is the same and has the same base condition in both cases, but the difference is the readiness, that is, what percentage of your maximum performance can you achieve at a specific time.

Physical examples are very illustrative and easy to relate to, but they are analogous to cognitive-based activities. Consider, for instance, the case of a senior sales person who travels from the EU to the US to close a deal. When the meeting comes, it is not the usual working hours for the salesperson and e.g. their sleepiness is high and the alertness and reaction time are low; as a result, the salesperson is not able to quickly and properly answer the questions of the potential customers and the deal is lost (low performance due to low readiness, which resulted in performing at only 40% of their capabilities). Nevertheless, the very same person in the same meeting could have closed the deal if it had been scheduled at a different time of the day (which would have resulted in the sales person performing at 70% of their capabilities, which would have been enough to close the deal).

A different situation would be if, instead of the senior sales person travelling to the US, a junior sales person based in the US had handled the meeting. The junior person may perform at their best, at 100% of their capabilities but still not be able to close the deal because their base condition is too low. In such a case, the base condition is too low to achieve the desired performance and this is not something that can be changed within a few days or weeks (it may take years for the junior person to gain enough experience to close the deal, in other words, to increase their base condition).

To summarise, the base condition defines the maximum performance that one can achieve at a certain activity; the base condition can be trained and improved. The readiness determines the percentage of your theoretical maximum performance (given by the base condition) that you can achieve at a certain event, given your actual body functions at the time that the event takes place. Using an analogy with water storage in a bucket, where the goal is to store as much water as possible: the base condition is the size of the bucket (e.g. 1 litre, 3 litre or 10 litre) and the readiness the fill level (e.g. 10%, 50% or 90%). A small bucket (3 litre, i.e. low base condition) completely filled (100%, i.e. high readiness) may store more water (3 litres of water stored) than a large bucket (10 litre, i.e. high base condition) that is only partially filled (10%, i.e. low readiness, thus 1 litre of water stored).

Personal States: In the context of this disclosure, a personal state preferably refers to a physiological state (associated with the body, physical), a psychological state (associated with the mind, mental, cognitive) or a combination thereof of a person, which can influence the outcome that one can achieve at a certain activity; in other words the user performance. Examples of personal states are sleepiness, alertness, body temperature, attention, vigilance, reaction time, selective attention, executive function, long-term memory, short-term memory, social cognition, perceptual and motor functions, cognitive performance, cognitive fatigue, physical fatigue. In other words, a determined or predicted performance score as disclosed in the context of the present invention can for example pertain to any of sleepiness, alertness, body temperature, attention, vigilance, reaction time, selective attention, executive function, long-term memory, short-term memory, social cognition, perceptual and motor functions, cognitive performance, cognitive fatigue, physical fatigue, as all these parameters or states affect user performance. Some of the personal states can be or are affected by the circadian rhythm.

Most personal states cannot be directly measured nor quantified (objective measurement), but they have a direct effect on the person, who feels them (subjective perception). For instance, a person unmistakably notices sleepiness (subjective perception), but quantifying how sleepy one feels (objective measurement) is more challenging; likewise, there is no device that can measure (and quantify) sleepiness. Another example is cognitive fatigue after an exam: a student may clearly *feel mentally tired* after an exam, but it may be difficult for the student to quantify how tired they are or how much rest they need to properly take another exam.

The relevance to daily functioning of different personal states depends on the use case. For instance, vigilance and reaction times are particularly important for a lorry driver, whereas the executive function may be more important for a business person. There are many factors that can affect personal states, including fatigue, hunger, sleep deprivation, physical health (like illness), medication, affective states (e.g. stress, anxiety, mood), as well as digesting a thick meal or alcohol.

Against this background, the present invention pertains to a computer-implemented method for improving user performance of at least one user at at least one defined point in time in the future, comprising at least one of the steps:
a) Acquiring data relating to a cognitive and / or physiological state and / or behavioural state of the at least one user,
b) Determining based on the data acquired in step a) an indication of a predicted user performance score at the defined point in time, preferably by calculating a numerical performance score,
c) Comparing the predicted user performance score to a desired user performance score, and
d) If the predicted user performance score indicates a predicted user performance falling short of a desired user performance indicated by the desired user performance score,
e) Selecting at least one activity and / or at least one time point or time range for performing an activity for adjusting the predicted user performance score to the desired user performance score or a tolerance range surrounding the desired user performance score, and
f) Outputting at least one prompt to the user prompting the user to perform the at least one selected activity and / or to perform an activity at the at least selected one time point or time range,
g) Wherein the user is, preferably continuously, monitored with regard to their cognitive and / or physiological state and / or behavioural state and / or the completion of the selected activity and / or the performance of the activity at the at least one selected time point or time range, and
h) the selection in step e) is, preferably continuously, updated based on the monitoring data obtained in step g) to, preferably continuously, tailor the selection to the user's detected behavior.

Data relating to a cognitive state preferably mean data capturing a state of the user's mind and for example pertain to alertness, attention, concentration, emotion (fear, anger, happiness etc.), stress, tiredness etc.

Data relating to a physiological state preferably mean data capturing a state of the user's body and for example pertain to heart rate, blood pressure, pupil dilation, skin conductivity, blood oxygenation, body temperature, skin temperature etc. The data relating to the physiological state can be obtained by any suitable sensor e.g. via a camera (photoplethysmogram), thermometer, pressure sensor etc.

Data relating to the behavioural state of the user preferably mean data capturing an aspect of user behaviour. Examples of data relating to the behavioural state of the user can be e.g. activity data tracking movement / activity of the user, location data capturing travelling activities by the user, screen time capturing e.g. sleep time of the user etc. The data relating to the behavioural state can be obtained by any suitable sensor e.g. via a gyroscope or GPS location tracker.

Additionally or alternatively, data relating to the surroundings of the user can be taken into account, e.g. air temperature, ambient noise level, air pollution level, etc.

Preferably, the defined point in the future is a time point specifically set by the user and preferably is not inferred by a time shift derived from a change in location of the user.

In other words, the present invention is configured to allow a user to set the defined time point in the future irrespective of a day-night-cycle at a given location.

The present invention is thus not limited in assisting a user in adjusting for jet lag by shifting their circadian rhythm, e.g. by setting a goal of a user performance of 90 % at 9 a.m. CET to 9 a.m. CST, but allows the user to set a defined time point in the future at which the desired user performance is to be achieved completely freely, e.g. by setting a goal of a user performance of 90 % at 16 p.m. CET (e.g. for a scheduled meeting) instead of 9 a.m. CET. This offers the user flexibility in setting the defined time point and desired user performance and makes the present invention more versatile.

According to the present invention, based on the data acquired in step a) an indication of a predicted user performance score at the defined point in time is determined, preferably by calculating a numerical performance score.

Preferably, the indication of a predicted user performance score at the defined point in time is determined based on a current state of the user and any detected scheduled activities of the user without any recommendations or adaptations proposed by the method.

For example, a predicted numerical user performance score of 65 % at the defined point in time of 8:00 am on Monday can be calculated based on the user's current physiological state "exhausted after running 20 km" and the scheduled activity "birthday party" starting on Sunday night at 22:00 pm.

The method can then select an activity such as "rest for 4 hours starting at 12:00 am Saturday" and / or select a time point of 19:00 pm and / or a time range of 19:00 pm to 23:00 pm for the scheduled activity "birthday party" for adjusting the predicted user performance score of 65 % to the desired user performance score of e.g. 90%.

The indication of a predicted user performance score preferably indicates the user score as a numerical value, percentage, stepped scale etc. For example, the predicted user performance score can be quantified according to a scale, e.g. 0-10 gauge, 3-level gauge high/medium/low, 2-level gauge yes/no, 0-1000 gauge or as a floating-point number.

A degree of confidence predicted user performance score can be included, wherein the degree of confidence is preferably expressed as a value (e.g. a number between 0 and 100, a 2-level gauge high/low, etc.) or as a range of values.

The predicted user performance score can also be a range, such as 80%-90%.

Thus, a predicted user performance score can be output as "Number + Confidence Indicator" (e.g. a score of 80 % with a confidence degree of 95 %) and / or as "Range + Confidence Indicator" (e.g. a score between 80 % - 90 % with a confidence degree of 99 %).

A user performance score can be predicted according to the present invention for any time point in the future, the past or the present. The term "predicted" can thus be understood as "estimated" or "inferred".

The "performance" of the predicted user performance score and / or desired user performance score can pertain to any personal state of the user and can capture e.g. cognitive performance, alertness-sleepiness, hunger, physical performance, etc. Thus, the "performance" can be measured in any desirable way defined by the user. In other words, the user can optimize any desirable parameter using the present method. For example, the user can set a desired performance score of 80 % alertness or 75% maximum force or 89 % maximum endurance.

A method according to the present invention can further comprise a calibration step that can comprise at least one standardized activity that the user is prompted to complete. The calibration step can be used to detect a user's performance level or scale (e.g. a value corresponding to 100 % performance) that can be helpful in calculating a relative performance score, such as a score of 80 %. The calibration process can involve a number of exercises or tests to be performed e.g. under different conditions, to extract a number of parameters to personalise a general or un-personalised model of a state, e.g. a cognitive state in terms of alertness-tiredness as assesses e.g. by a questionnaire or a physical fitness level in terms oxygen consumption VO₂ for that user.

The calibration step can be used to tailor a general performance pattern, such as high in the morning, dip after lunch, rise in the afternoon and then decline in the evening, to a specific user. For example, one particular user may be unexpectedly alert after 21:00 pm. The calibration can thus be used to generate a user-individual model of user performance, e.g. based on a wave form, preferably taking into account a circadian state or rhythm of the user.

Thus, the scaling step can comprise starting from a normalised waveform and scaling it according to the calibration values; wherein the calibration values may be obtained by asking the user to engage in a specific activity (cognitive and/or physical) and collect inputs based on e.g. sensors and manual user inputs.

Furthermore, a method according to the present invention can comprise a step of determining and / or reporting, possibly on-demand, a consequence associated with a predicted performance score. E.g. for a predicted sleepiness of 70 %, a probability of falling asleep in 20 min and an expected length of sleep of 8 hours could be determined and / or reported to the user. This can make the information regarding the user performance score more understandable or tangible to the user as it conveys the meaning of the user performance score in the real world.

The data acquired in step a) preferably comprise physiological data obtained via at least one sensor, and preferably further comprise data relating to scheduled events obtained from an electronic calendar of the user and / or manual user inputs, in particular self-report data.

The data acquired in step a) can also comprise activity data, screen time, location data, data inferred from device / smartphone use etc.. The data in step a) can comprise sensor data including raw data like accelerometer values or a photoplethysmogram signal and/or processed data like pulse rate or number of hours sleep at each sleep stage extracted from the raw data and / or automatic data, such as data pertaining to events derived from scheduled activities in a calendar such as meetings, events derived from emails like travel plans, manual user inputs (manual user entries, confirmation of suggestions) and / or inferred activities, e.g. the method automatically infers an activity that the user may have performed, especially when data is missing, possibly with a retrospective confirmation by the user.

A method according to the present invention can further comprise a step of determining an actual and / or individual circadian phase state of the user taking into account a detected circadian phase of user and / or detected chronotype of the user. For example, the actual and individual circadian phase state of the user can be used to determine the predicted user performance score.

Determining an actual and individual circadian phase state of the user can lead to a more accurate determination of the user performance score.

For example, when alertness is a parameter of performance to be considered, a general pattern in the whole population following a day-based time or schedule (sleeping during the night, activities during the day) could be as follows: Alertness is high, e.g. at 80 %, in the morning, dips to 50 % after lunch, rises in the afternoon after 15: 00pm to 80 % again and then steadily declines in the evening to 20 % at 23:00 pm. Determining an actual and individual circadian phase state of an individual user takes into account that individuals can deviate from this pattern and also that daily form and past activities (e.g. heavy partying on the weekend) can affect a user's phase state. According to an embodiment, the circadian phase of user and / or the chronotype of the user is detected to determine the user's actual circadian phase state, e.g. it can be determined that this individual user has an actual circadian phase state of an alertness of 80 % after lunch, because of a light meal and intriguing conversation during lunch.

Thus, by determining an actual circadian phase state for the user, preferably in the form of an absolute numerical value (such as 80% alertness or 75% peak muscle strength) instead of merely relying on an inferred circadian phase state of the user (inferred e.g. by taking into account a change in location, due to jet lag of 6 hours the user is inferred to have an alertness of 50 % instead of 70%) the actual circadian state of the user can be determined more accurately. For example, the user may unexpectedly feel high alertness, because they were not in synchronisation with the day-night-cycle at their starting location, so the change in location better aligns their circadian rhythm with the day-light-cycle.

Thus, in the context of the present disclosure, determining an actual circadian phase state of the user preferably means detecting for a specific point in time at which point on a recurrent curve indicating a personal state of a circadian-based process, such as alertness, the user is. In other words, the actual circadian phase state can give an indication about the amount the individual's circadian rhythm has to be delayed or advanced to align the user's circadian rhythm e.g. with a day-night-cycle ("Shifting the curve"). Further, determining an actual circadian phase state of the user preferably means determining a phase difference between a day-light-cycle (preferably at the user's location) and the user's actual circadian rhythm (recurrent curve indicating a personal state of a circadian-based process).

Further, in the context of the present disclosure, determining an individual circadian phase state of the user preferably means changing the shape of the recurrent curve indicating a personal state of a circadian-based process, such as alertness. In other words, determining the individual circadian phase state acknowledges that individual's circadian rhythms can deviate from the pattern of the general population (unpersonalised model or average behaviour).

The determined actual and / or individual circadian phase state of the user can be used to predict a user performance score at the defined point in the future. In other words, by determining a current state of the user it is possible to more accurately predict the user's performance in the future. The determined actual and / or individual circadian phase state of the user and the predicted user performance score at the defined point in the future preferably are calculated as quantified numbers. The quantified numbers can be expressed in a relative way (e.g. as a percentage, 80 %) preferably after a calibration step has been performed to gage an individual user's performance scale, e.g. to determine the individual user's 100 % or on a scale.

Preferably, according to the present invention, the actual and individual circadian phase state of the user is determined without inferring the circadian phase state of the user based on a time shift derived from a change in location of the user.

This distinguishes an embodiment of the present invention from an alternative strategy in that the actual circadian phase state for the user is not determined, but merely a time shift is determined by that the user's circadian rhythm is to be shifted regardless of the actual circadian phase state for the user.

In other words, according to an embodiment of the present invention, the actual circadian phase state is a specific absolute value capturing a performance of the user rather than a shift in circadian rhythm required (i.e. + 6 hrs, without determining the actual circadian phase state).

The determined actual and / or individual circadian phase state of the user can be used to derive a required adjustment of circadian phase or circadian rhythm of this individual user (e.g. + 6 hours) for adjusting the predicted user performance score to the desired user performance score or a tolerance range surrounding the desired user performance score.

For example, the actual and / or individual circadian phase state of the user at a given point in time e.g. 8:00 am can be at 50% alertness with the circadian rhythm /phase of the user indicating in the next 3 hours a rise to 80 % at 11:00 am. From this, it can be derived that if the user desires a performance score of 80 % alertness at 8:00 am on a day in the future, the circadian rhythm / phase of the user has to be shifted - 3 hours, so that the performance of 80 % is reached already at 8:00 am.

If, on the other hand, no actual and / or individual circadian phase state of the user is determined and e.g. a general prediction model based on the whole population is used according to that alertness is high, e.g. at 80 %, in the morning at 8:00 am, dips to 50 % after lunch, rises in the afternoon after 15: 00pm to 80 % again and then steadily declines in the evening to 20 % at 23:00 pm, it would be derived that the circadian rhythm / phase of the user has to be shifted not at all. This would not be accurate for this individual user who actually has an actual alertness of only 50 % at 8:00 am.

Thus, determining an actual and / or individual circadian phase state of the user can improve accuracy.

The determined actual and / or individual circadian phase state of the user can thus be used to determine whether the circadian phase of this individual user is to be advanced or delayed for adjusting the predicted user performance score to the desired user performance score or the tolerance range based on detected chronotype of the user and / or depending on an absolute temporal shift required, in which case the adjustment requiring less absolute temporal shift is selected.

The absolute temporal shift preferably means the amount of time, i.e. 1(-6)1 or |(+6)| both is an absolute temporal shift of 6 hrs.

As the circadian rhythm of the user is of periodic or recurrent nature, it is generally possible to shift a user's circadian rhythm forwards or backwards in time to achieve a certain circadian phase state and / or performance score at a desired point in time. In most cases the absolute temporal shift required is different for the backward and forward shift. For example, a backward shift of 3 hours or a forward shift of 21 hours (= 24 hours-3 hours) of the circadian rhythm of the user can be used for adjusting the predicted user performance score to the desired user performance score or the tolerance range. In this case, preferably, the backwards shift of 3 hours is selected, because the absolute temporal shift of 3 hours is smaller than the absolute temporal shift of 21 hours required for a forward shift.

Alternatively, a detected chronotype of the user can be taken into account when determining whether the circadian rhythm is to be shifted forward or backward in time. For example, a "night owl" type can more easily adjust to an adjustment backwards in time (e.g. going to be at 2:00 am instead at 23:00 pm) and a "early bird" type can more easily adjust to an adjustment forwards in time (e.g. getting up at 5:00 am instead of at 8:00 am).

Step e) can comprise selecting from a pool of activities, such as resting, physical activity, sleeping or cognitive exercise, an activity or course of activities predicted to adjust the predicted user performance score to the desired user performance score or a tolerance range surrounding the desired user performance score.

In other words, in step e) a course of action or a program can be generated that is predicted to bring the user performance score e.g. from a value of 80 % to a value of 85 % matching a desired user performance score of 85 %.

The tolerance range surrounding the desired user performance score can preferably be set by the user and can be e.g. + / - 10 %, preferably, +/- 5 %, in particular +/- 2 %.

Step e) can also comprise automatically updating an electronic calendar of the user to reschedule events and / or activities , wherein the user preferably can confirm the rescheduling of events and / or activities. If "events" and "activities" are mentioned it is to be understood that at least one event and / or at least one activity is meant.

Step e) can also comprise selecting for at least one scheduled activity identified in an electronic calendar of the user at least one alternative time point for performing that activity, after querying a data base whether the user has marked the at least one scheduled activity as amenable to rescheduling or not.

For example, according to the method an alternative time point of 21:00 pm can be selected for the activity "sleeping" scheduled at 23:00 pm. Because not all activities can freely be rescheduled, such as picking up the kids from school, shopping during shop openings hours, meeting other people at agreed time points, prior to selecting at least one alternative time point (or time range) for the at least one activity, a data base is queried whether the user has marked the at least one scheduled activity as amenable to rescheduling or not. This allows the user to defined fixed activities or events and activities or events that can be rescheduled. If no such information regarding a certain activity can be retrieved from the data base, a method or system according to the present invention can prompt the user to provide or enter such information.

According to an embodiment, a method according to the invention can comprise a step of prompting the user to input the desired user performance score for at least one defined point in time in the future or multiple time points in the future, wherein the user can preferably define the desired user performance score as a desired minimal numerical performance score at a single point in time or as a desired minimal cumulative numerical performance score summed over or averaged over multiple points of time.

This allows the user to set an optimization target for user performance. For example, the user can set a desired user performance score for at least one defined point in time in the future, e.g. an alertness level of 98% in a time range of between 8:00 am -10: 00 am for taking an important exam. This example illustrates the general point that the "at least one defined point in time in the future" as used in this disclosure can also mean "at least one defined time range in the future".

Instead of setting a defined desired user performance score the user can also set the desired user performance score to maximum performance score to ensure maximum user performance at the desired point in time regardless of the actual possible numerical performance score.

Generally, a system or method according to the present invention can select additional activities or also select activities not to be performed / skipped. Thus, scheduled activities can be recommended to be skipped.

Alternatively, the user can set a desired user performance score for multiple defined points in time in the future, e.g. an average alertness level of 85% in a time range of between 8:00 am -10: 00 am for a first meeting, a time range between 12:00 and 14:00 pm for a second meeting and a time range between 15:00 and 19:00 pm for a third meeting.

Furthermore, a method according to the present invention preferably contains a step of monitoring the user to check whether the user adheres to the selected course of action / activities. Actually checking whether the user follows the advice provided by the method and adjusts their behaviour to optimize user performance allows the method to updated the selected course of action / activities.

According to an embodiment, in step g) the user is, preferably continuously, monitored with regard to their completion of the selected activity and / or the performance of the activity at the at least one selected time point or time range, and it is determined whether the user has fully, partially or not completed the selected activity and / or whether the user has fully, partially or not completed the selected activity at the selected time point or time range.

For example, it is monitored whether the user completes the activity "Sleeping 2 hours from 12:00 to 14:00 pm" fully (2 hours), partially (e.g. 1 hour) or not at all (0 hour, 0 min) and whether the user performs the task at the at least one selected time point or time range, e.g. sleeps 2 hours from 11:00 am to 13:00 pm (the user has partially completed the activity at the selected time range), sleeps 2 hours from 12:00 am to 14:00 pm (the user has fully completed the activity at the selected time range) etc. The monitoring can rely on sensor data, but can also comprise manual input from the user, prompting the user to provide manual input or inferred data inferred e.g. from the user's habits or a recommendation output by the method or system.

Preferably, if sensor data are available, sensor data are used for monitoring. If no sensor data are available, preferably manual inputs by the user (e.g. after a prompt) are used for monitoring. If the user does not provide any input, data is inferred.

The detected circadian rhythm of the user (and preferably the user's detected actual and / or individual circadian phase state) is preferably updated based on the monitoring data capturing the actual user behaviour in the fashion of a closed-loop operation.

Similarly, the recommendations output by the system or method accoding to the present invention ( e.g. the activities selected to be performed or skipped at selected time points or ranges) are preferably also updated based on the monitoring data capturing the actual user behaviour in the fashion of a closed-loop operation.

A completion score can then be calculated to reflect a degree to that the user has completed the selected activity and / or to that the user has adhered to the selected time point or time range, wherein the completion score is preferably used in step e) to continuously tailor the selection to the user's detected behavior, in particular to update a scheduled plan of selected activities.

In the example above, the completion score can be calculated as follows:
"Sleeping 2 hours from 12:00 to 14:00 pm" fully (2 hours, completion score 100 %), partially (e.g. 1 hour, completion score 50 %) or not at all (0 hour, 0 min, completion score 0 %). This can be regarded as a quantitative completion score.

A temporal completion score can be calculated to measure whether the user performs the task at the at least one selected time point or time range, e.g. sleeps 2 hours from 11:00 am to 13:00 pm (the user has partially completed the activity at the selected time range, completion score 50 %, because 1 hour is performed in the selected time range), sleeps 2 hours from 12:00 am to 14:00 pm (the user has fully completed the activity at the selected time range, completion score 100 %) etc.

At least one quantitative completion score and at least one temporal completion score can be combined, preferably by multiplication to give a completion score reflecting a degree to that the user has completed the selected activity and / or to that the user has adhered to the selected time point or time range.

The completion score can be used to tailor the selection of activities to the user's detected behavior, in particular to update a scheduled plan of selected activities output to the user in step f). For example, the selection of activities can be updated in a closed-loop operation.

The output in step f) can be regarded as recommending to the user a course of action predicted to adjust the predicted user performance at the time point in the future to the desired user performance at this time point.

In the selection step e) multiple activities over multiple days can be selected and / or multiple activities within one day can be selected. This allows for long-term planning and performance optimisation but also for last minute performance optimisation at short notice.

A method according to the invention can comprise a step of determining whether a desired user performance score can be achieved with multiple activities within one day and, if this is determined not to be possible, the method can automatically select multiple activities over multiple days to adjust the predicted user performance score to the desired user score.

A method according to the invention can comprise a step of including user input in selecting at least one activity and / or at least one time point or time range for performing an activity. For example, multiple options can be output to the user and the user is prompted to select one option.

For example, the user can be asked, what would you prefer to do, activity A at time tA or activity B at time tB? The user may also introduce restrictions like a minimum performance at other activities. For example, adding one activity can have spreading consequences for any other scheduled activities that are updated accordingly.

Preferably, for each selected activity, a recommended start time and a recommended length or end time can be selected, possibly an intensity as well.

For example, activities for a within-day optimization of user performance can be selected in such a way to ensure that the activities in the same day do not limit the performance (e.g. due to accumulated fatigue).

The selected at least one activity can be a cognitive exercise and the selected time point can be before a scheduled activity, such as an important meeting. A cognitive exercise can be an exercise to improve a user's cognitive state, for example a brief meditation to help the user focus better or a breathing exercise the reduce anxiety and stress etc.

The selected at least one activity can be a cognitive exercise and the selected time point can also be after a scheduled activity, such as an important meeting. This can be seen as a cool-down cognitive exercise after an activity and can be useful for improving or keeping up performance over subsequent meetings or more days.

The selected at least one activity can be a sleep or rest activity (for example, for a given day, e.g. the method can output a recommended amount of sleep and wake-up time).

The selected at least one activity can also pertain to nutrition and comprise e.g. dietary proposal to ensure a desired performance at an activity. For example, the method can output a certain amount of food or calories that are easy to digest to avoid sleepiness and / or output a recommendation relating to coffee ingestion/avoidance, alcohol avoidance, etc.

The selected at least one activity can be a physical activity and the method preferably outputs to the user instructions regarding intensity, duration and moment during the day.

In addition to selecting activities / time points or ranges, the method can also use the predicted user performance score at the defined point in time to provide the user with advice regarding their behaviour at the defined point.

For example, in the context of the last meeting in a day full of meetings the method can provide the following advice: due to cognitive fatigue, some problems may seem overwhelming and you may not be able to find solutions that you would normally find; try not to dismiss the offer and instead ask for more time to analyse it and come back to them.

Furthermore, the method can comprise a step of outputting to the user an indication of feasibility of adjusting the predicted user performance score to the desired performance score. The indication of feasibility can be associated with one particular course of action comprising selected at least one activity and / or time point / range. This allows the user to make an informed choice between different options of courses of action.

The step f) of outputting at least one prompt to the user prompting the user to perform the at least one selected activity and / or to perform an activity at the at least selected one time point or time range can include outputting a recommendation to the user comprising rescheduling a planned/expected activity and / or scheduling a new activity (not planned/expected) and / or cancelling a planned/expected activity.

The recommendation can include changes to the schedule prior and / or posterior to the defined point in time at that the desired user performance is to be achieved.

The recommendation can include recommending time slots or scheduling adjustments to the user, based on a given time range indicated by the user, the predicted user performance at that time range and preferably additional constraints provided by the user.

For example, when the method is providing a recommendation relating to scheduling some activities / events considering a different time zone but with some freedom to schedule (e.g. morning or afternoon), the recommendation can be generated considering the desired user performance score at the defined point in time but also the disruption on the other planned/expected activities.

In the selection step e) it can be taken into account that the at least one time point or time range / time slot fulfils certain conditions (e.g. respect the local working hours) and minimises the effort required to achieve the targeted performance (this can mean e.g., minimising the number of activities required, achieving the targeted performance without compromising other activities).

For the scheduled activities, the user can provide an indication for each activity specifying whether that activity can be rescheduled (to any other time, no restrictions), can only partially be rescheduled (the user must specify a range of allowed shift values) or cannot be rescheduled. Preferably, the user enters such an indication via an input unit and the indication is stored in a data base that can be queried by a method or system according to the present invention.

In step f) next to the prompt and / or recommendation of course of action, an indication of feasibility can be reported indicating the feasibility of reaching the desired user performance score when the user complies with the prompt and / or recommendation.

For example, the indication can be indicated as "feasible" (the targeted value can be achieved whilst fulfilling all given restrictions of e.g. can-shift activities, minimum value for states in certain activities or moments in time, etc.), "possible" (it is possible to achieve the targeted value, but one or more of the given restrictions cannot be met) or "not possible" (it is not possible to achieve the desired user performance score).

Furthermore, a method according to the present invention preferably contains a step of displaying to the user a preferably continuous first curve indicating the predicted user performance in the future, preferably over the course of days, and / or displaying to the user a preferably continuous second curve indicating a detected actual user performance in the past, preferably over the course of days, wherein the first and second curves are preferably continuous with each other.

In other words, the user preferably is being presented with a continuous curve indication user performance in the past (actual detected user performance score and / or actual / individual circadian phase state) and in the future (predicted user performance score and / or predicted circadian phase state). An example of such a curve can be seen in the figures.

A method according to the present invention can comprise a step of outputting the predicted user performance score at the defined point in time and / or the desired user performance score to the user, wherein the predicted user performance score is preferably output as a continuous curve spanning a time interval in the past, the present and a time interval in the future and / or as numerical performance score.

This output allows the user at a single glance to comprehend whether the predicted user performance score matches the desired performance score or how close the predicted user performance score comes the desired performance score. An example of such a display can be seen in the figures.

A method according to the present invention can comprise a step of displaying or outputting information regarding a day-night cycle at a given location preferably set by the user, and / or scheduled future events and / or detected past events and / or acquired data relating to a cognitive and / or physiological state and / or behavioural state of the user in the past together with the predicted user performance score and / or desired user performance score.

This output allows the user to at a single glance comprehend their situation, e.g. will I wake up in the middle of the night at a new location, will I be able to focus in a meeting at 9:00 am etc.

Other aspects pertaining to the outputting step f) and the interaction with the user are described as follows:
The predicted user performance score is output to the user for at least one defined point in time (past, future, present) preferably as plot / curve over time. Alternatively or additionally, an actual (e.g. measured or inferred) user performance score is output to the user for at least one defined point in time (past, present) preferably as plot / curve over time. A degree of confidence can be displayed in any of the plots / curves. The degree of confidence can be output as a dashed line.

Curves of predicted user performance scores can be output for different scenarios in the same plot, e.g. two extreme cases corresponding to two scenarios can be displayed: scenario 1 where the user takes no special action, scenario 2 the user complies with all prompts / recommendations of the method. This display can motivate the user to comply with the prompts / recommendations generated by the method.

The time of at least one location can also be included in the output, preferably the current user's time zone, preferably also a second time zone if a trip to a different time zone is scheduled.

Also, a day-night cycle, preferably adjusted based on the actual user location (e.g. from GPS data, IP address or WIFI) or from a scheduled location (e.g. travel) can be included in the input. An example of such an output can be seen in the figures.

Planned events (e.g. calendar events, planned activities, recommended activities) can also be integrated in the output.

A method according to the present invention can be used for multiple users simultaneously.

For example, the method steps are, preferably simultaneously, performed for multiple different users and are preferably performed by a single device for the multiple different users to optimize user performance for all or at least some of the multiple users at at least one common defined point in time in the future.

This can be useful, for example, if a meeting is scheduled and all participants should achieve a desired performance score of at least 80 % at the meeting. Once the defined time point of the meeting has been set and the participants have been entered, the method then outputs a recommended course of action for each individual participant for this participant to achieve the performance score of at least 80 % at the meeting.

The desired performance score can be set by one user for all or a subset of the multiple users. For example, a chairperson of the meeting can set a desired performance score of at least 80 % at the meeting for all participants. Also, each can have their own desired performance score for the defined point in time, e.g. physical performance for user 1 is set to 60% and physical performance for user 2 is set to 80%.

A method according to the present invention can comprise a step of prompting a single user to input for all users of the multiple different users a common desired user performance score for at least one defined point in time in the future or multiple time points in the future.

The user can preferably define the common desired user performance score as a desired minimal numerical performance score for each of the multiple users at a single point in time or as a common desired minimal cumulative numerical performance score summed over or averaged over multiple points of time for each of the multiple users.

Based on the common desired user performance score, for each of the multiple users an individual plan of scheduled activities is generated based on the selection in step e). In other words, each individual user receives an individual course of action / recommendation to reach the desired user performance score.

Alternatively or additionally, the recommendation can consider more than one user at the same time.

Preferably, the defined time point is the same for all users, e.g. a common meeting for all the users. The defined time point can be set by a user and / or a recommendation of the scheduling of the defined time point can be recommended by the method or system.

For example, the method can select a time point or time range for the defined time point (e.g. a meeting) based on the predicted user performance score of all or a subset of the users (e.g. select a time when at least half of the participants in the meeting have a predicted performance score of 80%).

Each user can have their own specific recommendation / selected course of action / activities to meet the desired user performance score at the defined point in time. For example, each user can have their own specific requirements and / or circumstances (e.g. user 1 has a seven-hour train trip three hours before the meeting, user 2 has a one-hour meeting two hours before the meeting) that are taken into account.

The steps pertaining to the display of data and interaction with a user disclosed above can also be applied to multiple users. For example, these steps can also be performed for multiple users at once. For example, multiple curves / plots for multiple users can be displayed in a common graph or image. Multiple plots (also referred to as curves) can be displayed next to each other. The plots can be vertical-aligned plots and / or synchronised on the time axis, so that they preferably all move synchronously when scrolling. When displaying plots / curves of multiple users in a single graph, the plots / curves can be colour-coded to distinguish different users.

At least one scheduled and/or an expected activity per user can be displayed.

A waveform of a desired or predicted user performance score (e.g. alertness-tiredness, physical strength etc.) per user can be displayed. Data for each user can be displayed selectively.

Generally, the selecting step e) can comprise running and / or analyzing several simulations each linking a given activity and / or a time point of an activity to a predicted user performance score. For example, the results of these simulations can be in a data base. For example, one simulation indicates that sleeping at 8:00 am leads to a performance score of 80 % at 8:00 am and another simulation indicates that sleeping at 23:00 am leads to a performance score of 60 % at 8:00 am. Preferably, the activity and / or time point of the activity is selected predicted by the simulation to be optimal in adjusting the predicted user performance score to the desired user performance score. In this example, sleeping at 20:00 pm would be selected.

Another aspect of the present invention pertains to a device configured to perform a method according to the present invention. All features, elements and effects disclosed above in the context of the method equally apply to a device according to the invention and are not all repeated merely for the sake of avoiding redundancy. A device according to the present invention can be configured and / or programmed to perform any method step or set of method steps disclosed above in the context of the method.

Furthermore, it should be noted that the present disclosure is not limited to the combinations of features, effects and elements explicitly disclosed but that all features, effects and elements disclosed herein can be combined as desired and also claimed in isolation as desired.

A device according to the present invention is configured for performing a method according to one of the preceding claims for at least one user, and comprises:
a) a data acquisition unit configured for acquiring data relating to a cognitive and / or physiological state and / or behavioural state of the at least one user,
b) a prediction unit configured for determining based on the data acquired in step a) an indication of a predicted user performance score at the defined point in time, preferably by calculating a numerical performance score,
c) a comparison unit configured for comparing the predicted user performance score to a desired user performance score, and
d) a selection unit configured for, if the predicted user performance score indicates a predicted user performance falling short of a desired user performance indicated by the desired user performance score, selecting at least one activity and / or at least one time point or time range for performing an activity for adjusting the predicted user performance score to the desired user performance score or a tolerance range surrounding the desired user performance score,
e) an output unit configured for outputting at least one prompt to the user prompting the user to perform the at least one selected activity and / or to perform an activity at the at least one selected time point, and
f) a monitoring unit configured for, preferably continuously, monitoring the user with regard to their cognitive and / or physiological state and / or behavioural state and / or the completion of the selected activity and / or the performance of the activity at the at least one selected time point or time range, wherein the monitoring unit is configured to provide feedback to the selection unit, so that the selection of the selection unit is, preferably continuously, updated based on the monitoring data obtained by the monitoring unit to, preferably continuously, tailor the selection to the user's detected behavior.

For example, the device can be smartphone running a smartphone application that reports personalised recommendations to a user, mainly based on automatic data (and possibly some manual inputs). The device is preferably configured for an estimation / prediction and the quantification of a personal state, in particular a user performance, in time (past, present and future), from user data (automatic sensor data, manual inputs, data fetched from external services), and for outputting recommendations that are provided based on the data to achieve a certain goal. Typically the application / smartphone can rely on data such as sensor data and optionally manual inputs as input, then it can infer the past, present and future state or value of personal states, in particular user performance, quantifies these values them and reports the quantified value in time to the user. A device according to the present invention can be useful for recommending activities to achieve a user-specified goal, proposing an alternative schedule for an activity so that the performance at multiple activities can be simultaneously further optimised, etc. Apart from a smartphone, the invention may be implemented in a computer, tablet or any other device that can be programmed.

A device / system and method according to the present invention can also be configured to operate completely devoid of manual or conscious user input. In this case the user does not have to make a conscious / manual input and data is acquired e.g. via sensors and / or extracted from electronic calendars such as automatic data.

A device according to the invention can comprise an input unit configured for the user to input the defined point in the future as a time point specifically set by the user that is preferably not inferred by a time shift derived from a change in location of the user.

The data acquired by the acquisition unit can comprise physiological data obtained via at least one sensor, and preferably further comprise data relating to scheduled events obtained from an electronic calendar of the user and / or manual user inputs, in particular self-report data.

A device according to the invention can comprise an evaluation unit configured for determining actual and individual circadian phase state of the user taking into account a detected circadian phase of user and / or detected chronotype of the user.

The actual and individual circadian phase state of the user is preferably determined without inferring the circadian phase state of the user based on a time shift derived from a change in location of the user.

The evaluation unit can be configured for using the determined individual circadian phase state of the user to derive a required adjustment of circadian phase of this individual user for adjusting the predicted user performance score to the desired user performance score or a tolerance range surrounding the desired user performance score.

The evaluation unit can be configured for using the determined individual circadian phase state of the user to determine whether the circadian phase of this individual user is to be advanced or delayed for adjusting the predicted user performance score to the desired user performance score or the tolerance range based on detected chronotype of the user and / or depending on an absolute temporal shift required, in which case the adjustment requiring less absolute temporal shift is selected.

The selection unit can be configured for selecting from a pool of activities, such as resting, physical activity, sleeping or cognitive exercise, an activity or course of activities predicted to adjust the predicted user performance score to the desired user performance score or a tolerance range surrounding the desired user performance score.

If multiple activities or course of activities / actions are available, the selection unit preferably bases the selection on a trade-off between adjusting the predicted user performance score to the desired user performance score or a tolerance range and causing minimal disruption to a user's planned or scheduled events.

The selection unit can be configured for selecting for at least one scheduled activity identified in an electronic calendar of the user at least one alternative time point for performing that activity, after querying a data base whether the user has marked the at least one scheduled activity as amenable to rescheduling or not.

The output unit can be configured for prompting the user to input the desired user performance score for at least one defined point in time in the future or multiple time points in the future, wherein the user can preferably define the desired user performance score as a desired minimal numerical performance score at a single point in time or as a desired minimal cumulative numerical performance score summed over or averaged over multiple points of time.

The monitoring unit can be configured for, preferably continuously, monitoring the user with regard to their completion of the selected activity and / or the performance of the activity at the at least one selected time point or time range, and is further configured to determine whether the user has fully, partially or not completed the selected activity and / or whether the user has fully, partially or not completed the selected activity at the selected time point or time range.

The monitoring unit can be configured to calculate a completion score calculated to reflect a degree to that the user has completed the selected activity and / or to that the user has adhered to the selected time point or time range, wherein the completion score is transmitted to the selection unit and is used by the selection unit to continuously tailor the selection to the user's detected behavior, in particular to update a scheduled plan of selected activities.

A device according to the present invention can be configured for performing a method according to the present invention for multiple users simultaneously.

A device according to the present invention can also comprise an input unit configured for prompting a single user to input for all users of the multiple different users a common desired user performance score for at least one defined point in time in the future or multiple time points in the future, wherein the user can preferably define the common desired user performance score as a desired minimal numerical performance score for each of the multiple users at a single point in time or as a common desired minimal cumulative numerical performance score summed over or averaged over multiple points of time for each of the multiple users.

The selection unit can be configured for generating based on the common desired user performance score for each of the multiple users an individual plan of scheduled activities (also referred to as a course of action/ activities).

Yet another aspect of the invention pertains to a computer program product, comprising instructions which, when the program is executed by a device, cause the device to perform a method according to the present invention.

In the following, the invention is further illustrated by a description of use cases of embodiments of the invention.

For example, a user plans a business trip abroad, to a different time zone. The user is following a jet-lag mitigation plan that includes sleeping in the plane, but is unable to sleep in the plane. When the user arrives at the destination, the user guesses that they are jet-lagged but (a) they do not know by how much (how many hours) they are jet-lagged and (b) they do not know what to do to mitigate it. Conventional jet-lag mitigation plans do not provide further advice after landing and also do not update their recommendations if the user does not complete suggested activities, such as sleeping on the plane.

According to an embodiment, the present invention can report to the user a plot of the user's inferred alertness-sleepiness curve over time (in other words, an estimated quantified value of a personal state in the past, present and future based on the measured user behaviour). The plots preferably directly address (a) and in particular determine e.g. the amount of jet lag or the estimated cognitive performance at the targeted activity in the future. Furthermore, the estimations of the personal states are preferably continuously updated based on the measurements and aiming at meeting the desired performance and thus the recommendations output to the user are updated, which addresses (b). User performance scores can be estimated / predicted for different personal states such as "alertness", "physical strength", "concentration" etc. A personal state can be regarded as a performance score, because a performance score captures a specific personal state of a user.

The report can include an indication of the user's expected productivity, per scheduled activity, or per hour, per kind of activity, etc.; e.g. based on the local or destination time at the origin or destination. The report can comprise displaying a quantified number (e.g. in a 0-10 gauge), or can be more qualitatively like indicating high/medium/low. The user might then want to revisit their planned activities to include some more resting activities, in view of their expected performance considering the recent lack of sleep.

Another example pertains to a business trip abroad from A to B, with A and B having different time zones. The user has only one meeting to attend at B. A conventional jet-lag mitigation plan is not advisable because the user needs need to be cognitively highly performant at the specified time at B and only at that time (e.g. meeting is in the afternoon at B, ideal is to participate as if the user is jet-lagged so that the afternoon at B is their morning based on time A).

In this scenario, a quantification and visual representation of the personal state (e.g. an alertness state as captured by a performance score) aids in understanding the situation. The method or system according to an embodiment recommends (giving a few options) a plan to, for example: (a) achieve a minimum degree of performance in each indicated meeting (i.e. for each meeting i, the expected performance is at least X_i), (b) maximise the combined performance in all indicated meetings (maximising the sum of the expected performance in all indicated meetings).

In another example, the user is following a jet-lag mitigation plan but is not able to follow all recommendations. For instance, the plan recommends sleeping between 00:00 and 8:00 and the user sleeps between 01:00 and 8:30. Conventional jet lag mitigation plans do not allow indicating partial activity completions or completion of different activities.

Also, conventional jet lag mitigation plans do not monitor whether the user actually follows the recommendations and completes the recommended activities. In contrast, according to an embodiment of the invention, the system or method continuously adjusts both the predicted performance score and the recommendation comprising the selected activities and time points based on sensor data and / or inferred data and / or user input (can be prompted), which measures the actual behaviour of the user.

Another example pertains to a university student who after two consecutive days with exams has one week until the next one; the student does not know whether they should take a one-day rest after the two exams to be better recovered or instead spend the day studying.

According to an embodiment of the invention, the system or method can recommend whether or not to take a rest day, based on the accumulated fatigue and scheduled exam; also considering that studying is needed and it requires achieving a certain cognitive performance level.

In another example, a user is planning a short business trip abroad. The user does not know at what times the meetings at the destination should be scheduled, because the user will not be equally productive throughout the entire day due to jet lag and tiredness.

According to an embodiment of the invention, the system or method can select for the user the time points / ranges of the day where the user will be most productive upon arrival, showing (and recommending) in a calendar the best times to schedule meetings.

Different recommendations can be output for the future by the system or method, considering different scenarios of activities performed by the user and different optimization targets, e.g. maximum expected performance in a single meeting, maximise the sum of expected performances in a number of meetings, at least a minimum expected performance in each of the meetings.

Each recommendation can consider a targeted or usual schedule at the destination, such as the usual working schedule at the destination E.g. from Barcelona, where meetings start at 9:30 to Seattle (+9h) where meetings start at 7:30 (-2h) only +5h are to be compensated. Thus, additional information regarding the locations or destinations can be taken into account, information only regarding a time zone at the starting point and destination is not enough, typical working hours are be considered as well in this example.

In another example, a user is travelling abroad. A conventional jet-lag mitigation plan recommends sleeping in the plane. But depending on whether the user travels first class or economy class, it may be more or less likely that they can sleep on the plane.

According to an embodiment of the invention, the system or method can adapt the recommendations based on the likelihood that the user can comply with the recommendations. E.g. if they won't easily sleep on the plane, the recommendation makes the user board the plane more tired (e.g. by selecting a vigorous physical exercise prior to departure to the airport), so that the user can sleep more easily on the plane. This is an example in that the invention takes into account automatic data, e.g. from an electronic calendar of the user indicating the flight class, to fine-tune the selecting and / or outputting step.

The system or method can show different scenarios, depending on different actions that the user may take, such as sleeping in the plane vs not sleeping in the plane.

Preferably, at least part of the data used to create the recommendation is captured automatically e.g. based on phone usage or calendar (scheduled) events. The automatic inference can include chronotype, frequent go-to-bed time, sleep duration, wake-up time, usual working hours etc.

The system or method can also suggest some habit changes before the trip, e.g. the recommendation can also comprise information regarding when to schedule activities (e.g, meetings but also exercise) before the trip.

The system or method can consider the impact of its output recommendation on a local schedule at a given location in order to adapt to the desired schedule. For example, the recommendation can be to instead of delaying the schedule, advance it because the local meetings early in the morning are mandatory. This is particularly relevant for a short trip with one important meeting at the destination, as there are usually two peaks of alertness in a day of a user.

According to another example, a meeting is already scheduled at the destination and the user wants to participate in the meeting at their best, but at the same time minimise the amount of days spent abroad and the amount of habit changes (to minimise interference with their regular work).

According to an embodiment of the invention, the system or method can schedule a custom plan including some changes of habits a few days before the trip and habit adaptation at the destination.

In another example, a meeting is scheduled outside the usual working hours (e.g. a meeting with a remote partner in a different time zone). The recommendation also applies, but without the time difference due to travelling. Thus, the invention is useful not only for mitigating jet lag but also for optimising performance without a change of location.

According to an embodiment of the invention, the system or method can automatically shift or reschedule meetings and set new appointments or only suggest shifting rescheduling them.

In another example, as part of a job interview, a user is invited to a meeting wherein the user needs to quickly react to stimuli; the meeting is in the morning. The user wants to perform at their best (fastest reaction time) in that meeting.

According to an embodiment of the invention, the system or method can provide a quantified estimation of the user's reaction time (in other words a predicted user performance score) throughout the day, including during the job interview.

The system or method can also output a recommendation to shift the entire reaction time curve so that the meeting falls within the maximum. In other words, the circadian phase or rhythm of the user can be shifted.

In another example, the user has underperformed in an activity and does not know why, but wants to prevent this from happening again.

According to an embodiment of the invention, the system or method can offer a visualisation of past activities and user performance scores, to learn what was the performance score in the past and understand why they underperformed or over-performed in an activity. E.g. if the alertness score was in the past always only 35% at 15:00 pm after lunch, the reason the user did badly in a meeting might be that the meeting was at 15:00 pm after lunch.

In another example, a Dutch person and a Greek person are in a relationship and they are trying to schedule a video call. Since they speak in English to each other and yet none of them are native English speakers, they need to be cognitively attentive so that the language does not constrain neither the messages or feelings that they want to express nor their capacity of understanding the partner.

According to an embodiment of the invention, the system or method can consider both users simultaneously, including their specific activities, and recommend a time that optimises the cognitive performance for both of them, e.g. each of them achieving at least a minimum performance score -specific per user or same for both of them-, or maximising the performance score combined of both of them.

The system or method could in this example be used individually by one of the user or by both of them, to help the users agreeing on a time to schedule the call.

According to another example, a group of friends want to schedule a football match and a lunch during a weekend. Some of them are coming from abroad, from a different time zone, some others are shift workers and some others are not. They do not know when to schedule the match, morning or evening, but they want all to be physically fit to minimise the risk of an injury

According to an embodiment of the invention, the system or method can be used to quickly identify the best moment to schedule the match, considering that some of them may be jet lagged (trip, shift workers) and the particular chronotype and usual schedule of each of them. Furthermore, this invention can also propose personalised changes (e.g. activities, timing of activities) shortly before the match to optimise the physical readiness of each of them.

In another example, a company with multiple locations is scheduling a get-together event in a single location; employees from a number of different time zones will come together for a few days, but some of them will be jet lagged.

According to an embodiment of the invention, the system or method can recommend the most convenient times to schedule activities, as well as specific activities for each individual to adjust their scheduling to the activities.

At a higher level, according to an embodiment of the invention, the system or method can also be used to identify the best location out of all company locations, so that the disruption on the everyday activities of all employees is minimised.

As illustrated by the preceding exemplary use cases, the present invention can help to overcome the following exemplary problems:
People often seek to optimise their performance in their professional and personal activities. For instance, one may want to perform at their best in a meeting with investors right after a long-distance trip, or to be more productive on a regular basis to achieve a promotion (professional life); likewise, an amateur runner may want to complete a marathon or improve their personal best for personal satisfaction (personal life). Either way, optimising one's own performance on specific activities is often sought.

When planning and preparing for an activity in the future, users face the challenge of ensuring that they will perform at their best at the targeted activity. Especially for cognitive-based activities such as a meeting or an exam, users can often plan a few days or weeks ahead (namely short term); whilst this time range is not enough to substantially change the base condition, it is long enough to tune up readiness even if the targeted activity is preceded by a disruption such as a long-distance trip. In other words, a proper short-term tune up maximises the likelihood that one will perform at their best given their base condition. Unfortunately a proper tune up involves a twofold challenge, as the factors that determine readiness (personal states such as sleepiness or cognitive fatigue) cannot always be directly measured or quantified despite the fact a person can feel them (in other words, a person may feel e.g. very sleepy when sleepiness is high -subjective perception-, but precisely quantifying it -objective measurement- is not possible), and, even if measuring and quantifying them were possible, it would still be necessary to predict their value in the future for the targeted activity and consider the expected activities in between.

In summary, when a user wants to perform at their best (ensure high readiness) at a targeted activity scheduled in the short-term future, they face the twofold challenge of (a) determining whether a special tune-up preparation is required (i.e. they are unable to measure readiness and to predict its value in the future) and (b), if a tune up is required, how to achieve it (help is needed to optimise their performance).

Conventional applications providing recommendations to the user (a plan with a number of activities suggested, that may help in achieving a certain goal) do so in an openloop manner. That is, the plan is not adjusted according to the actual activities carried out by the user, nor do these applications report the current / actual and the predicted states of the internal body processes.

It would be very advantageous for activity planning and performance optimisation to infer the state of a process/state, quantify its value and report it to the user; in this way a person could better understand their current state, in particular pertaining to their performance, and they could choose to adjust their processes/states to optimise their performance. It would also be beneficial to predict the value of the process/states in the future.

This is currently not possible according to the prior art or it is too inaccurate and thus users are not able to (a) determine whether they need to adjust their processes/states to perform at the desired level nor (b), should an adjustment be required, implement the adjustment (in other words, they do not know what to do to achieve this adjustment). There is a need to assist users in planning, preparing and optimising their performance in future activities. The present invention can contribute to address this need.

Further features, aspects, elements and effects of the present invention become apparent from the subsequent description of the invention in other words and by taking reference to the figures.

Generally put, the present invention pertains to both hardware and software elements, e.g. the hardware of a smartphone comprising a unit that is configured / programmed in a specific way. The hardware elements can include a device for communicating with the user including means for displaying information to the user, such as a screen, and means for acquiring information from the user, such as a keyboard or a touchscreen (such a device can be a smartphone); and a device equipped with sensors to monitor the user (for example, the same smartphone already includes sensors; regardless of that, wearables with sensors can monitor the user and connect to the smartphone for sharing data). A device according to the invention does not have to comprise sensors. A processing unit is required, which is often part of the device for communicating with the user, and a communications unit to fetch and/or send data from/to external resources such as a remote device.

The software elements of the present invention can include both backend software and frontend software implemented in a control unit. The backend software preferably processes the input data and provides values that express the quantified value of a state of the user, for example a user performance score; in other words, the backend software preferably processes the user data and other data used and analysed in the steps of a method according to the invention. The frontend software preferably displays the data such as performance scores, recommendations etc. determined by the backend software and preferably allows the user to provide direct inputs to the backend software. The frontend software and the backend software can operate independently of each other.

The hardware elements comprise sensors, which can be built-in the same device used for interacting with the user (e.g. a smartphone) or external, such as those found in smartwatches, rings or any other wearable, as long as the external sensors are able to communicate with the main device. Sensors are very useful in the context of the present invention. For example, sleep monitoring can be much improved by sensors. If no external sensors are available, the sleep pattern can be inferred from the usage of the smartphone (e.g. the longest period without interactions including the night corresponds to sleeping, then the last prior interaction and the first posterior interaction define the go-to-bed and the wake-up times respectively). This simple method is unable to determine the actual number of hours that the user slept during the night (as some users may not fall asleep right after going to bed, or they could wake up a few times during the night). Instead, a smartwatch comprising sensors can properly detect not only the number of hours that the user spent in the bed, but the actual amount of hours that the user slept.

Having reliable sensor data is helpful in making accurate estimations. A sensor used in the context of the present invention may also include a processor to pre-process the sensed data and provide processed data to the software elements, e.g. a smartwatch, instead of providing raw accelerometer data and raw photoplethysmogram (PPG) data, can process them and provide sleep data in the form of hours of light sleep, hours of deep sleep, hours of REM sleep, hours awake, average heart rate at each sleep stage, average breathing rate at each sleep stage, the sequence of sleep stages that the user followed during the night, etc. In the context of this invention, a sensor is preferably any device that can acquire information from the user, such as a camera, an electrocardiogram (ECG), an accelerometer, a glucose meter, etc.

Regarding the software elements of the present invention, two software elements are further described below: the backend software and the frontend software. Both software elements communicate to each other and exchange information, but they can also operate independently from each other. Broadly speaking, the backend carries out all calculations and the frontend outputs the values (calculated by the backend) to the user and allows the user to provide inputs; in other words, the frontend is responsible for user interaction whereas the backend is responsible for the calculations.

The backend software quantifies and estimates (and / or predicts) a value of a state, such as a user performance score for the past, present or future. The estimated value (e.g. the user performance score) can refer to a point in time in the future, in the past or the present; in other words, the backend preferably quantifies and estimates the value that the state will have in the future, that it had in the past or that it has right now. The backend software can comprise a model. The backend model can provide a list of recommend activities (including the type of activity, a start time, an end time and optionally activity characteristics such as an intensity), typically to meet a target (e.g. a desired user performance score) whilst fulfilling a number of restrictions. An example of a target may be achieving a minimum value for the quantified value of a state at a user-specified time.

The state value quantification and estimation (e.g. the prediction or estimation of a user performance score at a defined point in time) is carried out by a model, which preferably relies on "*user data*" and "*common data*" as inputs. "*user data*" preferably means data that is specific to that user and that the model may use to estimate the value of the state; this includes, amongst others, data explicitly provided by the user (e.g. manual introduction of sleep times and perceived quality of sleep), data fetched from external resources (e.g. entries in the user's electronic calendar, data from an external database such as an Electronic Medical Record - EMR) and data acquired by sensors or external devices. Likewise "*common data*" preferably means any other data, not specific to any individual in particular, that the model may use to estimate the value of the state; an example of common data is an un-personalised model or a baseline behaviour that is common for a certain group of individuals.

Depending on the use case, the model may not rely on all the sources of data to compute the estimations; some data sources may not be available or they may not bring valuable insights. For instance, sleep can be derived from a planning in the calendar (e.g. planned sleep between 23:00 and 07:00), from manually-entered data (e.g. reported sleep between 23:30 and 07:00), from sensor data (e.g. measured sleep between 23:51 and 07:03) or it can even be guessed (e.g. from common data and based on planned/measured activities the same day and the day before); note that inferring sleep from a past estimation (e.g. copying the same sleep pattern than exactly one week ago), eventually relies on one of the estimations described earlier. Usually relying on sensor data (which often provide automatic and highresolution measurements) yields more accurate estimations than those based on, solely, guessed activities, planned activities, or manually-entered data. If there is redundancy in the data, the model may discard some of the data (e.g if sleep data is available from both a sensor and an event in the calendar, the model ignore the calendar event and process only the sensor data).

Sensors can monitor all the activities carried out by the user, regardless of whether they were planned or not; this is especially relevant for users following an activity plan, such as a weight-loss plan, a well-being plan or a therapeutic plan, as there are often mismatches between the planned activities and the actual activities carried out by the user; incorporating the actual activities to the evaluation, as opposed to relying on only the planned and targeted activities, often yields more accurate estimations.

Thus, according to an embodiment of the invention the method or device monitors user in step g) with at least one sensor to capture the user's behaviour preferably without the user having to consciously make any inputs. The monitoring step g) can comprise detecting additional, unexpected activities by the user (e.g. the user is scheduled to sleep at 21:00 pm, but heads to a party instead). Also, using a sensor or multiple sensors for monitoring user behaviour can resolve user behaviour differing from recommended behaviour, e.g. the planned activity is sleeping between 23:00 and 7:00, but the user sleeps between 00:30 and 8:10. This information can be feasibly and accurately captured according to the present invention with a sensor e.g. present in a smartwatch (sleep monitoring device).

A model used by the present invention can be implemented based on different techniques. A simple model can be implemented based on rules (rule-based model); a more complex model can be implemented based on machine learning (trained model) that preferably can also adapt itself according to the user behaviour. Next, a simple exemplary model for estimating the physical fatigue (a state) is described and an example of inferring chronotype is inferred.

One of the states of user characteristics that can be used to tune the recommendations according to the particularities/preferences/specifics/needs of each user is the chronotype. The chronotype can be directly queried from the user, it can be evaluated with a questionnaire but it can also be inferred from measured activities

As mentioned earlier, the measurements can be obtained from a smartphone (e.g. last interaction before going to sleep, first interaction after waking up, and go-to-bed and wake-up-times are identified by a long gap in user-based interactions and/or movement data coinciding with the user's usual sleep times) or from a wearable device, which may not only provide go-to-bed and out-of-bed times but also fall asleep and wake up times, as well as sleep information.

A simple method to infer the chronotype of a user is, for a user following a day-based schedule working from Monday to Friday, to analyse the go-to-sleep and wake up times in the weekend, when the person is free to plan their day: a morning-type person would go to bed before 22:15, an evening-type person would go to bed after 1:45 and the rest would be neutral. A more advanced model may consider how difficult is for the person to sleep, e.g. by measuring elapsed time between going to bed and falling asleep, how difficult is for the person to wake up, e.g. by measuring the elapsed time between waking up and getting out of the bed, monitoring activities carried out (planned or not planned), wake-up times, elapsed time between waking up and leaving the bed, whether an alarm is used or waking up before the alarm; likewise, the activity and sleep patterns during workdays may be used as well.

Having a reliable indication of the user's chronotype is advantageous to provide recommendations that the user can likely adhere to. E.g. for an evening-type person, it may be easier to delay the schedule than to advance it.

An exemplary model that can be used in the context of the present invention is configured to quantify the state *Physical Fatigue* (PF) of a user. The level of physical fatigue directly impacts user performance cognitive and physical) and thus estimating / predicting physical fatigue is a way of estimating / predicting user performance. The model in this example includes a variable, namely the Physical Readiness (PR) ranging from 0 (minimum) to 100 (maximum). The PF is defined as the complementary of the PR: PF = 100-PR

The exemplary model uses as inputs the indication of activities carried out by the user and the time interval wherein this activity took place; the PF is updated accordingly. If the updated value would exceed the range of PF, then PF saturates to its maximum or minimum value. This exemplary model only considers three different activities and it does not distinguish between different intensities for each activity. The only parameters are, besides the activity itself, the start and end times of the activity. The types of activities and their updates rates are:
- Physical activity - The user performs physical exercise, such as running, walking or biking. PF update rate of -10 units / hour
- Sleep - The user sleeps. PF update rate of +12,5 units / hour
- Other (default) - The user is neither sleeping nor doing a physical activity (e.g. sitting on a chair resting, standing still and reading a book, eating in a bar). PF update rate of -5 units / hour

The indications of activities performed by the user may be fetched from an external resource such as an electronic calendar, manually introduced by the user or provided by a sensor. The indication of the activity preferably describes, at least, the sort of activity, the start time and the end time. More advanced models may consider further states describing the activity, such as the intensity of the activity (which may be used by the model to provide a more accurate estimation) or variable update rates depending on the time of the day or the length of the activity. The indications of activities may be provided as activities that will be performed in the future, activities that have been performed in the past or as live updates of an ongoing activity; likewise, an indication of activity may be first provided and then updated (e.g. a planned workout, wherein the user reports after the workout how intense they felt it was).

Alternatively, instead of receiving the indications of activities as inputs, the indication of activities can be inferred from the known behaviour of the user. A simple approach is to assume that the user carries out the same activities like in a past day, such as the day before or one week before (exactly seven days before). More advanced activityinferring systems may be used, but their functionality in all cases is the same: to provide an indication of an activity that is likely that the user has carried out or will carry out. The inferred activity may include the same amount of characteristics than an actual activity (e.g. the start time, the end time, the nature of the activity or the intensity of the activity to mention a few examples).

This exemplary model of PF provides a quantified output of this state (an integer number, e.g. a user performance score) and it can be used to estimate the value of PF in the future (e.g. a predicted user performance score, typically based on planned activities and expected activities) and also in the past (e.g. a past user performance score, typically based on reported activities -calendar, sensors, manual inputs, etc.- and possibly reported activities), to let the user review the quantified value that the state had in the past. A value expressing the current quantified value of the state (e.g. a present or current user performance score) can be provided as well. Alternatively to the integernumber quantification, the output of the model could be quantified as a three-level variable (e.g. high, medium or low), or as a two-level value (e.g. high or low, yes or no) or as a floating-point number.

The model can also provide a confidence value next to the quantified state value. The confidence is another output of the model that expresses, according to the model itself, the likelihood that the actual state or actual user performance score (in this example, the actual PF or how tired the user is) matches the value reported by the model. The confidence can also be provided as a number (e.g. an integer in the 0-100 range) or as a discrete-level variable (e.g. high or low). The confidence can be evaluated in different ways; a simple possibility for this exemplary model is to label it as "high" if there is at least one activity measured from sensors or reported by the user that day, and "low" otherwise.

The parameters of the model can be pre-defined but they can also be adjusted (once or continuously). For example, a model with no parameters could be provided with an initial dataset from which an initial set of values for that user can be inferred (model calibration or personalisation); this initial dataset can be provided at once or it can be acquired over a certain period of time such as two weeks. Another possibility is to rely on a model that already has its parameters set; these set of parameters can be tuned according to the behaviour of the user (i.e. the model adapts to the user). Personalising the model often yields more accurate results for each user in particular than general (un-personalised) models. For instance, consider the example of sleep. In average, an adult needs about eight hours of sleep per day; however, when looking into particular individuals, some people may only need to sleep seven hours whereas others may need nine hours. Thus, depending on the characteristics of the user, eight hours of sleep may be more than necessary, exactly what is needed or less than necessary. Model personalisation or calibration is a well-known technique and used in many devices.

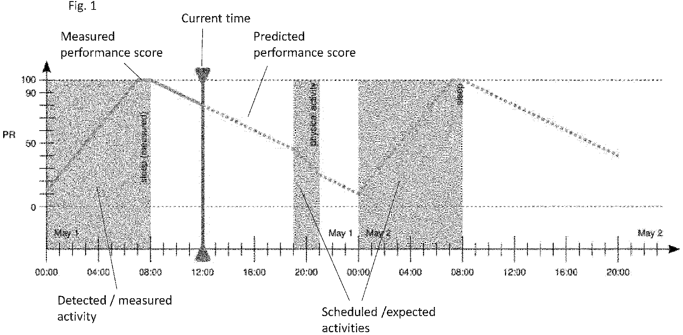

Figure 1 illustrates this model with an example. Fig. 1 shows a PR quantification and estimation based on the exemplary model described above as a backend software.

The vertical line indicates the current time. The activities to the right (in the future) of the vertical line are expected (based on e.g. the activities of one week ago or based on an electronic calendar). The activities to the left of the vertical line are activities in the past that have been detected e.g. based on sensor input or via a manual input by the user.

Fig. 1 shows the PR score (an example of a user performance score) in time, including both past and future times (the current time is indicated with a solid red line, at May 1^{st}, 12:00). To further emphasise the difference between the past and future PR scores (also referred to a value), the past values are drawn with a solid line whereas the future ones (predicted) are drawn with a dotted line. The scores are indicated as a continuous curve spanning past, present time and future. The example in Fig. 1 includes three activities: a measured activity (sleep) and 2 expected activities (physical activity and sleep). Based on the expected activities, future values of PR can be predicted. The continuous curve (solid or dotted) quantifies the PR (an example of the personal state and / or performance score), as the vertical axis indicates the values that it defines (e.g. the performance score scale) and thus each point of the curve corresponds to a value (performance score).

As an example of calibration, the update rates provided above could be the un-personalised model. However, over a period of two weeks sleep data may be collected (e.g. by automatic sensor data and manual inputs from the user); during this period the user sleeps, in average, 7 hours and the user indicates that they feel fully recovered. Then the model can be personalised for sleep, from 12,5 units per hour (12,5 x 8 = 100) to 14,3 units per hour (14,3 x 7 ~ 100).

The model of physical fatigue and physical readiness illustrated by Fig. 1 can be used to propose activities.

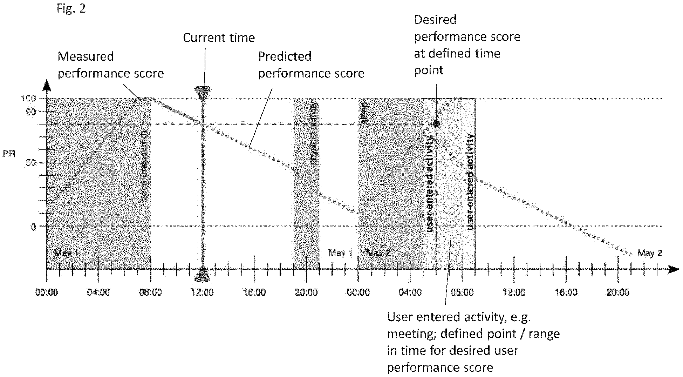

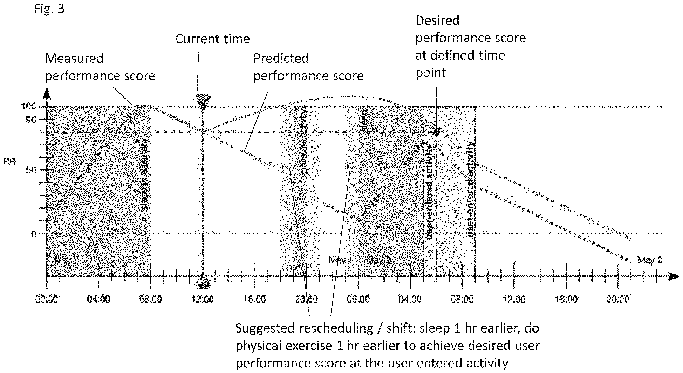

For instance, a user may specify that they want a minimum of 95% of physical readiness for an activity at 6:00 at a specific day (the usual sleep hours for this user are between 00:00 and 08:00), with at least 1h between sleep and the start of the activity. The model can use the expected activities to estimate the PR if the user carries on with their usual activities. If the estimation does not meet the required value, then a plan of proposed activities can be sketched. As an example, the following steps can be followed. These are illustrated in Fig. 1, 2 and 3.
1. Check if the expected PR is already within the range requested by the user (from figure 1). For example, in Fig. 2 a desired level of physical readiness (an example of a desired performance score) is indicated by a dashed horizontal line at 80 % physical readiness. The predicted physical readiness at the desired time point is compared with the desired level of physical readiness.
   1.1. If the predicted physical readiness at the desired time point matches the desired level of physical readiness, nothing needs to be changed. Yet, even in this case, this condition preferably is periodically checked as the actual behaviour of the user may change and the expected PR at the specified day and time may fall outside the requested range. Then the rest of the algorithm should be applied.
   1.2. If the predicted physical readiness at the desired time point does not match the desired level of physical readiness activities can be proposed to adjust the predicted physical readiness at the desired time point to match the desired level of physical readiness.
2. Given that the PR follows a recurrent behaviour based on the user's circadian rhythm or state, the desired level of physical readiness (also referred to as a target value) can be achieved by advancing or delaying the user's circadian rhythm or state . In this example, this decision is taken based on the number of hours (N) that the expected PR curve needs to be delayed (N>0) or advanced (N<0) before reaching the targeted value; in particular, based on the lowest number of hours required. Following figure 2, advancing the sleep time by 1h already meets the specified criterion; on the contrary, if delaying the schedule, more than 3h of shift will be required (approximately 13h are required) and thus a multi-day adjustment is necessary.
   2.1. If the chronotype of the user is known, a weighting factor may be used when evaluating either advancing or delaying the user's circadian rhythm or state and thus adjusting the user's scheduled events / activities. E.g. a weighting factor of 1.0 for neutral people, 0.8 for morning-type people and 1.2 for evening-type people can be used; then the evaluation is based on a cost, that is defined as the product of the number of hours times the weighting factor for positive shifts (advance the schedule) and the number of hours times the inverse of the weighting factor for negative shifts. In other words, the absolute temporal shift (e.g. 6 hours) can be multiplied with a weighting factor to take into account the chronotype of the user. Applied to the example shown in Fig. 1-3, if the person is a night type, advancing would cost 1.0h x 1.2 = 1.2, whereas delaying would cost 13.0h /1.2 = 10.8; 1.2 < 10.8 thus the decision would also be to advance by 1h.
   2.2. When advancing or delaying the schedule, this can be understood as moving the whole expected activities towards one direction, possibly limiting the shift per day to two or three hours max. Some activities can shift (like going to sleep, going to the gym) but there are some other activities that can only partially shift (e.g. start working at 8:00 instead of at 9:00 may be possible, but not earlier) or cannot shift (e.g. bringing the kids to the school and picking them, the school time is fixed). Can shift, can partially shift or cannot shift is something that may be specified by the user. In other words, the user can defined which activities are amenable to rescheduling and to what degree they are amenable to rescheduling. When setting a goal, there can be situations where it is not possible to meet the target whilst fulfilling all other requests; then this can be reported to the user, possibly with a recommendation of activities with some compromises (e.g. achieve a lower performance at the targeted activity, recommend to skip a gym session, recommend to add a gym session, etc.).
3. In the present example, a recommendation can be output to the user to go to sleep N hours earlier/later than usual to meet the target, as during sleep the PR increases. In this example, the recommendation output would be to sleep 1h earlier than usual, and change nothing else.
   3.1. A recommendation based on an alternative approach can consider how likely is that the user can sleep at the specified time. This can be derived from the physical fatigue as well. If the PR is higher than a certain threshold (e.g. 40), then it is very unlikely that the user may be able to sleep at that time (condition 1). A solution is then to split the shift of N hours over multiple days to ensure that condition 1 is fulfilled in all of them.
   3.2. Also, a recommendation based on an alternative approach can consider the side effects of the recommendation on the regular user activities. For instance, if the PR goes below 5, the user may be too tired to do their regular activities (condition 2). A solution is then to split the shift of N over multiple days to ensure that condition 2 is fulfilled in all of them.
      Also, prior and posterior activities surrounding the defined point in time can be taken into account, such as an athlete travelling to the place where a race starts or having breakfast prior to the race, which may impose further restrictions on the recommended activities.
4. The result of the recommendation is reported to the user. This is illustrated in Fig. 3 in that the scheduled sleep and physical activity are both shifted by 1 hour earlier (to the left in Fig. 3).
   4.1. If reaching the specified goal is *feasible* (*feasible* means in this example that all the conditions defined above -condition 1, condition 2, etc.- are met and that the targeted value can be reached), then the activity(ies) are proposed to the user. Note that this can involve activities in multiple days or within a single day.
   4.2. If reaching the specified goal is *possible* (*possible* means in this example that the targeted value can be reached but not all the conditions defined above are met), then the user can be notified of recommended activity(ies) with a warning that some conditions may not be met and the potential consequences of that; multiple recommendations can be provided and the user can be prompted to choose between a number of options.
   4.3. If reaching the goal is *not possible* (*not possible* means in this example that the targeted value cannot be reached), then the user is informed and, optionally, a recommendation can still be provided, to get the targeted state (e.g. the predicted user performance score) as close to the goal (e.g. desired user performance score) as possible.

The approach described in the previous examples is valid for both multiple-day recommendations but also for within-day recommendations.

An example of only within-day recommendations is described next.

Consider the state *Cognitive Fatigue* (CF) or its complementary *Cognitive Readiness* (CR), defined analogously as the *Physical Fatigue* (PF) and the *Physical Readiness* (PR):
- Both CF and CR range between 0 and 100
- CF = 100-CR

In a regular day (proper sleep during the night and without the user being previously in an unusual state that influences the user state at this day), CR starts at 80 in the morning and decreases to 20. During the day, the CR decreases at a rate; however, certain activities can modify this default pattern, for example:
- A cognitive-intense activity (e.g. an intense meeting): update rate of -10 units / hour
- Cognitive warm-up exercises: update rate of +60 units / hour, limited to maximum of 15 min every 2h; the units gained by these exercises decrease at a rate of at least 7 units / hour even without a cognitive-intense activity

- Cognitive relaxation exercises: update rate of +15 units / hour, limited to a maximum of 30 min and limited to immediately after a cognitive-intense activity
- Physical fatigue: PF < 40 reduces CR by 5 units of CR, PF < 20 reduces CR by 10 units of CR, PF < 10 reduces CR by 20 units
- Other activity: update rate of -3,75 units / hour

In an example, a user schedules at their calendar a 2h meeting and at 9:30 and marks it as important.

Even though there is in this example no specific goal in terms of CR, the model of the system / method recommends doing a 15min warming-up exercise at 9:10 to maximise the user's performance at the meeting. This will in this example add 15 additional units of CR so that the user will be able to perform better during the meeting (the decrease in the next 5min before the meeting starts is less than 1 unit); next to the recommendation, the invention may provide also information explaining the advantages of having a higher CR in a meeting.

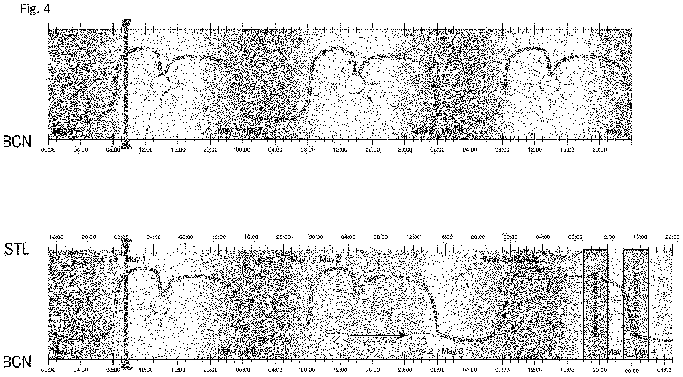

In the following, it is explained based on Fig. 4, how information may be output or displayed (also referred to as reported) to the user. The output unit and / or input unit comprises in this example a frontend software that is described above and is the element of the software of this invention that interacts with the user, for both reporting to the user and also for allowing the user to manually input data.

Data reporting in this example comprises representing in an easy-to-understand and visually-friendly way to the user the data evaluated by the backend software. Typically only a part of the backend-calculated data is presented at once; for instance the value of a state (e.g. a performance score) within a certain time range, or the value of two states (e.g. two performance scores pertaining to different personal states such as physical readiness and alertness) within a certain time range.

Preferably the presented data include some context information to ease their interpretation. For instance, in an example, a user plans a business trip to a destination with a different time zone for a meeting. When the user wants to learn about their expected cognitive performance at the scheduled meeting (namely, in a week's time), it is informative to display as well the local time at both the source and the destination, the daylight (at the source, at the destination and/or combined as e.g. what the user will experience) or the date at both source and destination. An example of such a display is given in Fig. 4.

These are a few examples of context information; in general context information includes any information that could be relevant for the user when interpreting the backend-calculated values, such as uncertainty ranges or multiple values depending on whether the user performs certain activities in the future.

The examples included in figure 1 to figure 3 already include some context information, such as the local time and date, measured / detected past activities, expected activities and planned activities.

Figure 4 includes two examples also showing the daylight based on the user location, e.g. Barcelona (BCN), for a use case wherein the user stays at the same place (see top panel) and for a case wherein a long-distance trip is planned (see lower panel), in this case to Saint Louis, MS (STL). In the latter case, the local time at both the starting point (BCN) and the destination (STL) is displayed as well.

Finally, further variants of the invention are disclosed as follows:
The embodiments listed above can include a simple rule-based model for the sake of clarity and simplicity of the explanation. The model could be more complex and even be based on machine learning (trained model), but in principle the same approach can be followed. Thus, in other words, according to the present invention, a machine learning model can be used that is configured to perform any of the method steps disclosed herein. However, if the model becomes too complex (especially when the model is trained), there could be a conceptual difference in the way of using it.

The model used in the embodiments is observable and understandable; this means that it is feasible to interpret the internal operations performed on each input and therefore translate the desired change in the output into changes in the input. Like the example in Figure 3, where it is very clear how each change in the input affects the output.

In other words, the starting point is the output (a targeted featured value or desired user performance score) and the target is propagated back and translated into requirements for other activities.

However, very complex models are not interpretable and they are often treated as a black box. This is typically the case of neural networks for object recognition in image processing (neural networks are a specific type of machine learning-based model), wherein the inputs to the model are the intensity of each pixel and the output is the probability of the image containing a specific object (e.g. a car). Whilst strictly speaking the model itself is not a black box (one can follow exactly the operations that are carried out by the model), the model is too complex to interpret the logic of the operations (in other words, understand why the model performs each operation, interpret the intermediate results); as a result, understanding how a change in a pixel increases or decreases the output is not feasible and therefore the only feasible way to know how a change in the input affects the output is to try it. Because of this reason, such complex models are often categorised as black boxes.

Since these systems are effectively black boxes, it is unfeasible to understand why these small changes cause such a change in the output; or, in other words, it is unfeasible to, starting from the desired outcome (e.g. 99% confidence assessing that image A contains a coffee mug), determine which pixels need to change in image A (and by how much) to achieve the desired outcome.

In the present case, if the model becomes so complex that it effectively behaves as a black box, the process for selecting and / or recommending activities can be based on simulations.

In other words, a number of different combinations of inputs (i.e. different sets of activities, of different length, different nature, at different times, of different intensities, etc.) would be first generated (at random or not, see below), then each of them would be tried or simulated and they would be sorted according to a criterion (typically whether the target (e.g. the desired performance score) is met or not, but it could also be how far below/above the target they are, or including the number of modifications compared to the regular schedule, etc.); the selected combinations of inputs would be presented to the user.

In this case the starting point is the input and its effect on the output is simulated. In other embodiments of the invention, the starting point is the targeted output and the desired change compared to the baseline level is translated into a sets of inputs based on the understanding of the model.

Leaving AI systems aside, black-box systems are not uncommon in the real world, especially when dealing with mechanical systems or systems with many variables. Different techniques have been developed to deal with them; in some cases, it is even possible to characterise the black box and approximate its behaviour with a simple and interpretable model.

In the context of this invention, this can mean that, when dealing with black-box-like models, the invention may also rely on well-known statistical and numerical methods, such as Monte Carlo or system identification.

Another variant of the present invention is that it could be used to target a different mental state, like an emotion. In this case, the performance score may pertain to happiness (e.g. 80% happy) , gratefulness, relaxation etc.

For instance, after a tough announcement at work, one may request to end the day in a better mood (e.g. happier than now); the recommendation may then include not only specific activities (e.g. read a book; do some physical exercise) but also define the content of the activity (e.g. recommend to read a book in particular; go to the swimming pool and follow training programme A).

To implement this variant effectively, it can be advantageous to have some further knowledge about the user (e.g. which activities makes the user happy, which could be learnt by asking the user to report their mood after an activity e.g. via a digital diary of the user), and preferably also to have access to resources designed for eliciting a certain reaction in the user (e.g. in the form of a library of activities designed to elicit a specific reaction).

Another variant of this invention relates to multiple people jointly trying to find the best time for all of them to schedule an activity.

The example discussed above refers to two people trying to be cognitively available, but it could involve more people and an activity of different nature (e.g. a group of friends, some of them coming from abroad, trying to schedule a football match - they all need to be physically and cognitively ready to minimise the risk of injuries).

In this variant, the data from all the users involved can be analysed together and the recommendation is based on all of them (and it is the same for all users). The more users are involved, the more likely it is that some conditions may not be fulfilled for some users; different criteria can be used in this case as well: require a minimum feature value (e.g. a minimum performance score of 70 % physical readiness) for all the users, maximise the aggregated value of the feature for all users, etc.

Each user may have their own specific restriction (whether the activities that have already been scheduled or are expected can be rescheduled or not, by how much, etc.).

Furthermore, the analysis can be carried out from a goal and rescheduling previous activities to meet that goal, or based on a number of simulations (different sets of inputs) and sorting them according to how much they comply with the target, or based on any other technique; regardless of the method, the result is in this example always a recommendation including the schedule for the targeted activity and changes (rescheduling, new activities, skipping or cancelling planned activities) prior to the targeted activity at the defined point in time and possibly changes (rescheduling, new activities, skipping or cancelling planned activities) after the targeted activity.

Aspects of a device according to the present invention can be summarised as follows:
A first aspect pertains to a device configured for performing a method according to the present invention for at least one user, comprising:
a) A data acquisition unit configured for acquiring data relating to a cognitive and / or physiological state and / or behavioural state of the at least one user,
b) a prediction unit configured for determining based on the data acquired in step a) an indication of a predicted user performance score at the defined point in time, preferably by calculating a numerical performance score,
c) A comparison unit configured for comparing the predicted user performance score to a desired user performance score, and
d) a selection unit configured for, if the predicted user performance score indicates a predicted user performance falling short of a desired user performance indicated by the desired user performance score, selecting at least one activity and / or at least one time point or time range for performing an activity for adjusting the predicted user performance score to the desired user performance score or a tolerance range surrounding the desired user performance score,
e) An output unit configured for outputting at least one prompt to the user prompting the user to perform the at least one selected activity and / or to perform an activity at the at least one selected time point, and
f) a monitoring unit configured for, preferably continuously, monitoring the user with regard to their cognitive and / or physiological state and / or behavioural state and / or the completion of the selected activity and / or the performance of the activity at the at least one selected time point or time range, wherein the monitoring unit is configured to provide feedback to the selection unit, so that the selection of the selection unit is, preferably continuously, updated based on the monitoring data obtained by the monitoring unit to, preferably continuously, tailor the selection to the user's detected behavior.

A second aspect pertains to a device according to the first aspect, further comprising an input unit configured for the user to input the defined point in the future as a time point specifically set by the user that is preferably not inferred by a time shift derived from a change in location of the user.

A third aspect pertains to a device according to the first or second aspect, wherein the data acquired by the acquisition unit comprise physiological data obtained via at least one sensor, and preferably further comprise data relating to scheduled events obtained from an electronic calendar of the user and / or manual user inputs, in particular self-report data.

A fourth aspect pertains to a device according to one of the first to third aspects, further comprising an evaluation unit configured for determining actual and individual circadian phase state of the user taking into account a detected circadian phase of user and / or detected chronotype of the user.

A fifth aspect pertains to a device according to the fourth aspect, wherein the evaluation unit is configured for using the determined individual circadian phase state of the user is to derive a required adjustment of circadian phase of this individual user for adjusting the predicted user performance score to the desired user performance score or a tolerance range surrounding the desired user performance score.

A sixth aspect pertains to a device according to the fifth aspect, wherein the evaluation unit is configured for using the determined individual circadian phase state of the user to determine whether the circadian phase of this individual user is to be advanced or delayed for adjusting the predicted user performance score to the desired user performance score or the tolerance range based on detected chronotype of the user and / or depending on an absolute temporal shift required, in which case the adjustment requiring less absolute temporal shift is selected.

A seventh aspect pertains to a device according to one of the first to sixth aspects, wherein the selection unit is configured for selecting from a pool of activities, such as resting, physical activity, sleeping or cognitive exercise, an activity or course of activities predicted to adjust the predicted user performance score to the desired user performance score or a tolerance range surrounding the desired user performance score.

An eighth aspect pertains to a device according to one of the first to seventh aspects, wherein the selection unit is configured for selecting for at least one scheduled activity identified in an electronic calendar of the user at least one alternative time point for performing that activity, after querying a data base whether the user has marked the at least one scheduled activity as amenable to rescheduling or not.

A ninth aspect pertains to a device according to one of the first to eighth aspects, wherein the output unit is configured for prompting the user to input the desired user performance score for at least one de-fined point in time in the future or multiple time points in the future, wherein the user can preferably define the desired user performance score as a desired minimal numerical performance score at a single point in time or as a desired minimal cumulative numerical performance score summed over or averaged over multiple points of time.

A tenth aspect pertains to a device according to one of the first to ninth aspects, wherein the monitoring unit is configured for, preferably continuously, monitoring the user with regard to their completion of the selected activity and / or the performance of the activity at the at least one selected time point or time range, and is further configured to determine whether the user has fully, partially or not completed the selected activity and / or whether the user has fully, partially or not completed the selected activity at the selected time point or time range.

An eleventh aspect pertains to a device according to the tenth aspect, wherein the monitoring unit is configured to calculate a completion score calculated to reflect a degree to that the user has completed the selected activity and / or to that the user has adhered to the selected time point or time range, wherein the completion score is transmitted to the selection unit and is used by the selection unit to continuously tailor the selection to the user's detected behaviour, in particular to update a scheduled plan of selected activities.

A twelfth aspect pertains to a device according to one of the first to eleventh aspects, wherein the device is configured for performing a method as recited in one of claims 1 to 17 for multiple users simultaneously.

An thirteenth aspect pertains to a device according to the twelfth aspect, further comprising an input unit configured for prompting a single user to input for all users of the multiple different users a common desired user performance score for at least one defined point in time in the future or multiple time points in the future, wherein the user can preferably define the common desired user performance score as a desired minimal numerical performance score for each of the multiple users at a single point in time or as a common desired minimal cumulative numerical performance score summed over or averaged over multiple points of time for each of the multiple users.

An fourteenth aspect pertains to a device according to the thirteenth aspect, wherein the selection unit is further configured for generating based on the common desired user performance score for each of the multiple users an individual plan of scheduled activities.

## Claims

1. A computer-implemented method for improving user performance of at least one user at at least one defined point in time in the future, comprising the steps:
a) Acquiring data relating to a cognitive and / or physiological state and / or behavioural state of the at least one user,
b) Determining based on the data acquired in step a) an indication of a predicted user performance score at the defined point in time, preferably by calculating a numerical performance score,
c) Comparing the predicted user performance score to a desired user performance score, and
d) If the predicted user performance score indicates a predicted user performance falling short of a desired user performance indicated by the desired user performance score,
e) Selecting at least one activity and / or at least one time point or time range for performing an activity for adjusting the predicted user performance score to the desired user performance score or a tolerance range surrounding the desired user performance score, and
f) Outputting at least one prompt to the user prompting the user to perform the at least one selected activity and / or to perform an activity at the at least selected one time point or time range,
g) Wherein the user is, preferably continuously, monitored with regard to their cognitive and / or physiological state and / or behavioural state and / or the completion of the selected activity and / or the performance of the activity at the at least one selected time point or time range, and
h) the selection in step e) is, preferably continuously, updated based on the monitoring data obtained in step g) to, preferably continuously, tailor the selection to the user's detected behavior.

2. Method according to claim 1, wherein the defined point in the future is a time point specifically set by the user and preferably is not inferred by a time shift derived from a change in location of the user.

3. Method according to claim 1 or 2, wherein the data acquired in step a) comprise physiological data obtained via at least one sensor, and preferably further comprise data relating to scheduled events obtained from an electronic calendar of the user and / or manual user inputs, in particular self-report data.

4. Method according to one of the preceding claims, further comprising a step of determining an actual and individual circadian phase state of the user taking into account a detected circadian phase of user and / or detected chronotype of the user.

5. Method according to claim 4, wherein the determined individual circadian phase state of the user is used to derive a required adjustment of circadian phase of this individual user for adjusting the predicted user performance score to the desired user performance score or a tolerance range surrounding the desired user performance score.

6. Method of claim 5, wherein the determined individual circadian phase state of the user is used to determine whether the circadian phase of this individual user is to be advanced or delayed for adjusting the predicted user performance score to the desired user performance score or the tolerance range based on detected chronotype of the user and / or depending on an absolute temporal shift required, in which case the adjustment requiring less absolute temporal shift is selected.

7. Method according to one of the preceding claims, wherein step e) comprises selecting from a pool of activities, such as resting, physical activity, sleeping or cognitive exercise, an activity or course of activities predicted to adjust the predicted user performance score to the desired user performance score or a tolerance range surrounding the desired user performance score.

8. Method according to one of the preceding claims, wherein step e) comprises selecting for at least one scheduled activity identified in an electronic calendar of the user at least one alternative time point for performing that activity, after querying a data base whether the user has marked the at least one scheduled activity as amenable to rescheduling or not.

9. Method according to one of the preceding claims, further comprising a step of prompting the user to input the desired user performance score for at least one defined point in time in the future or multiple time points in the future, wherein the user can preferably define the desired user performance score as a desired minimal numerical performance score at a single point in time or as a desired minimal cumulative numerical performance score summed over or averaged over multiple points of time.

10. Method according to one of the preceding claims, wherein in step g) the user is continuously monitored with regard to their completion of the selected activity and / or the performance of the activity at the at least one selected time point or time range, and it is determined whether the user has fully, partially or not completed the selected activity and / or whether the user has fully, partially or not completed the selected activity at the selected time point or time range.

11. Method according to claim 10, wherein a completion score is calculated to reflect a degree to that the user has completed the selected activity and / or to that the user has adhered to the selected time point or time range, wherein the completion score is used in step e) to continuously tailor the selection to the user's detected behavior, in particular to update a scheduled plan of selected activities.

12. Method according to one of the preceding claims, further comprising a step of displaying to the user a preferably continuous first curve indicating the predicted user performance in the future, preferably over the course of days, and / or displaying to the user a preferably continuous second curve indicating a detected actual user performance in the past, preferably over the course of days, wherein the first and second curves are preferably continuous with each other.

13. Method according to one of the preceding claims, further comprising a step of outputting the predicted user performance score at the defined point in time and / or the desired user performance score to the user, wherein the predicted user performance score is preferably output as a continuous curve spanning a time interval in the past, the present and a time interval in the future and / or as numerical performance score.

14. Method according to claim 12 or 13, comprising further displaying or outputting information regarding a day-night cycle at a given location preferably set by the user, and / or scheduled future events and / or detected past events and / or acquired data relating to a cognitive and / or physiological state and / or behavioural state of the user in the past together with the predicted user performance score and / or desired user performance score.

15. Method according to one of the preceding claims, wherein the method steps are, preferably simultaneously, performed for multiple different users and are preferably performed by a single device for the multiple different users to optimize user performance for all or at least some of the multiple users at at least one common defined point in time in the future.

16. Method according to claim 15, further comprising a step of prompting a single user to input for all users of the multiple different users a common desired user performance score for at least one defined point in time in the future or multiple time points in the future, wherein the user can preferably define the common desired user performance score as a desired minimal numerical performance score for each of the multiple users at a single point in time or as a common desired minimal cumulative numerical performance score summed over or averaged over multiple points of time for each of the multiple users.

17. Method according to claim 16, wherein based on the common desired user performance score for each of the multiple users an individual plan of scheduled activities is generated based on the selection in step e).

18. A device configured for performing a method according to one of the preceding claims for at least one user, comprising:
a) A data acquisition unit configured for acquiring data relating to a cognitive and / or physiological state and / or behavioural state of the at least one user,
b) a prediction unit configured for determining based on the data acquired in step a) an indication of a predicted user performance score at the defined point in time, preferably by calculating a numerical performance score,
c) A comparison unit configured for comparing the predicted user performance score to a desired user performance score, and
d) a selection unit configured for, if the predicted user performance score indicates a predicted user performance falling short of a desired user performance indicated by the desired user performance score, selecting at least one activity and / or at least one time point or time range for performing an activity for adjusting the predicted user performance score to the desired user performance score or a tolerance range surrounding the desired user performance score,
e) An output unit configured for outputting at least one prompt to the user prompting the user to perform the at least one selected activity and / or to perform an activity at the at least one selected time point, and
f) a monitoring unit configured for, preferably continuously, monitoring the user with regard to their cognitive and / or physiological state and / or behavioural state and / or the completion of the selected activity and / or the performance of the activity at the at least one selected time point or time range, wherein the monitoring unit is configured to provide feedback to the selection unit, so that the selection of the selection unit is, preferably continuously, updated based on the monitoring data obtained by the monitoring unit to, preferably continuously, tailor the selection to the user's detected behavior.
